(19) Europäisches Patentamt
European Patent Office
Office européen des brevets

(11) Numéro de publication: **0 074 912**
**B1**

# (12) FASCICULE DE BREVET EUROPEEN

(45) Date de publication du fascicule du brevet: **13.02.85**

(51) Int. Cl.[4]: **C 07 C 69/52**, C 07 C 67/38

(21) Numéro de dépôt: **82420096.8**

(22) Date de dépôt: **09.07.82**

(54) **Procédé de séparation du palladium des produits issus de la carbonylation des diènes conjugués en présence de palladium.**

(30) Priorité: **16.09.81 FR 8117463**

(43) Date de publication de la demande: **23.03.83 Bulletin 83/12**

(45) Mention de la délivrance du brevet: **13.02.85 Bulletin 85/7**

(84) Etats contractants désignés: **AT BE CH DE FR GB IT LI LU NL SE**

(56) Documents cités: **US - A - 4 269 781**

**Le dossier contient des informations techniques présentées postérieurement au dépôt de la demande et ne figurant pas dans le présent fascicule.**

(73) Titulaire: **RHONE-POULENC CHIMIE DE BASE, 25, quai Paul Doumer, F-92408 Courbevoie (FR)**

(72) Inventeur: **Jenck, Jean, 11, rue Lakanal, F-69100 Villeurbanne (FR)**

(74) Mandataire: **Varniere-Grange, Monique et al, RHONE-POULENC RECHERCHES Service Brevets Chimie et Polymères Centre de Recherches de Saint-Fons 85, rue des Frères Perret B.P. 62, F-69192 St-Fons Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 074 912 B1

BUNDESDRUCKEREI BERLIN

**Description**

La présente invention a pour objet un procédé de séparation du palladium des produits issus de la réaction de carbonylation par du CO des diènes conjugués en présence d'un alcool, d'un hydracide halogéné et d'un catalyseur au palladium.

Le principal avantage de la présente invention consiste en ce que le palladium séparé se trouve en solution alcoolique et peut être directement recyclé en vue d'une nouvelle opération de carbonylation tout en conservant son activité.

Il est connu d'après le brevet japonais n° 48.5564 de préparer des monoesters d'acides carboxyliques $\beta,\gamma$-insaturés, par carbonylation des diènes conjugués par de l'oxyde de carbone, en présence d'un monoalcool, d'un catalyseur au palladium non halogéné et d'un hydracide halogéné, à une température de l'ordre de 100°C et une pression d'oxyde de carbone de l'ordre de 100 bars; aucune information quant à une méthode de recyclage du catalyseur au palladium n'est donnée.

Le brevet américain n° 4.172.087 décrit la synthèse d'esters de l'acide pentène-3 oïque en mélange avec une quantité importante d'esters de l'acide nonadiène-3,8 oïque, par carbonylation du butadiène par de l'oxyde de carbone en présence d'un alcool, d'une amine tertiaire hétérocyclique, d'un halogénure de palladium complexé par une phosphine tertiaire monodentée ou d'un sel de palladium non halogéné complexé par une phosphine tertiaire polydentée; la présence d'une phosphine tertiaire monodentée et d'une amine tertiaire hétérocyclique permet de récupérer après distillation des esters de l'acide pentène-3 oïque et des esters de l'acide nonadiène-3,8 oïque, un liquide contenant le catalyseur au palladium; ledit liquide peut être recyclé vers le milieu à carbonyler. Un tel procédé est réalisé en l'absence d'hydracide halogéné; il est très peu sélectif en esters de l'acide pentène-3 oïque.

Le brevet américain n° 4.269.781 décrit la synthèse d'esters de l'acide nonadiène-3,8 oïque par cabonylation dimérisante du butadiène-1,3, c'est-à-dire par réaction du monoxyde de carbone et d'un alcool sur le butadiène en présence de palladium et d'un ligand donneur qui est un composé tertiaire d'un élément du groupe VB, en excluant toute présence d'halogénure coordiné au palladium métallique. Dans ce procédé on utilise comme ligand donneur une phosphine tertiaire, on conduit la réaction en présence d'eau, puis on met en contact le mélange réactionnel avec un solvant hydrocarboné (ou bien on conduit la réaction en présence d'un solvant hydrocarboné, puis on met en contact le mélange réactionnel avec de l'eau) pour former deux phases liquides, l'ester de l'acide nonadiène-3,8 oïque étant alors récupéré dans la phase hydrocarbonée et la phase hydroalcoolique, sensée contenir le système catalytique, est recyclée à la réaction. Dans ce procédé, il est essentiel d'opérer en présence d'une quantité contrôlée d'eau pour qu'il y ait une séparation de phases et de veiller à ce qu'il n'y ait pas de monoxyde de carbone en excés au stade de la mise en contact et/ou de la séparation de phases pour limiter les fuites de palladium dans la phase hydrocarbonée. Mais par un tel procédé on ne peut pas obtenir d'esters d'acides carboxyliques $\beta,\gamma$-insaturés avec un rendement satisfaisant.

L'objet de la présente invention est un procédé de séparation du palladium des produits issus de la réaction de carbonylation par de l'oxyde de carbone des diènes conjugués en présence d'un alcool, d'un hydracide halogéné et d'un catalyseur au palladium, réaction de carbonylation en vue de la synthèse d'esters d'acides carboxyliques $\beta,\gamma$-insaturés, ledit procédé étant caractérisé en ce que:

- les produits issus de la réaction de carbonylation sont mis en contact avec un sel d'onium quaternaire d'un élément du groupe VB choisi parmi l'azote, le phosphore et l'arsenic, un alcool polaire et un solvant apolaire hydrocarboné aliphatique ou cycloaliphatique présentant une constante diélectrique (mesurée à 20—25°C) inférieure à 2,3.
- et en ce qu'après décantation de la phase alcoolique et de la phase organique formées lors de l'opération de mise en contact, on récupère le palladium et le sel d'onium quaternaire dans la phase alcoolique et les produits issus de la réaction de carbonylation dans la phase organique.

On entend par »phase alcoolique« la phase dont le solvant est constitué par l'alcool présent lors de l'opération de mise en contact.

On entend par »phase organique« la phase dont le solvant est constitué par le solvant hydrocarboné apolaire présent lors de l'opération de mise en contact.

Parmi les sels d'onium quaternaires pouvant être mis en oeuvre, on peut citer ceux présentant un anion quelconque et un cation onium quaternaire répondant à l'une des quatre formules suivantes:

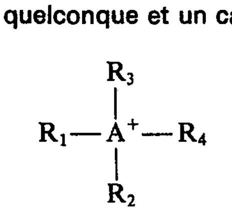

(I)

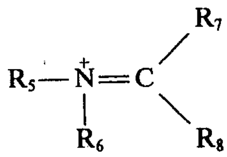

(II)

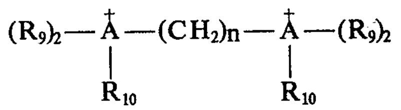

(III)

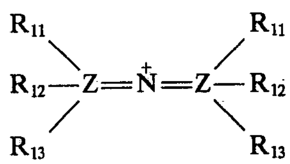

(IV)

où:

- A représente de l'azote, du phosphore ou de l'arsenic
- $R_1$, $R_2$, $R_3$, $R_4$ sont identiques ou différents et représentent:

  un radical alkyle linéaire ou ramifié contenant de 1 à 16 atomes de carbone, ledit radical alkyle étant éventuellement substitué par un groupe phényle, hydroxy, halogéno, nitro, alkoxy, ou alkoxycarbonyle;
  un radical alcényle linéaire ou ramifié contenant de 2 à 12 atomes de carbone, de préférence de 4 à 8 atomes de carbone, et tout particulièrement un radical alcényle dérivé du diène conjugué mis en oeuvre;
  un radical aryle contenant de 6 à 10 atomes de carbone, ledit radical aryle étant éventuellement substitué par au moins un groupe alkyle contenant de 1 à 4 atomes de carbone, alcoxy, alkoxycarbonyle, ou halogéno;
  deux desdits radicaux $R_1$ à $R_4$ pouvant former ensemble un radical alkylène, alcénylène ou alcadiénylène, linéaire ou ramifié et contenant de 3 à 6 atomes de carbone;

- $R_5$, $R_6$, $R_7$, $R_8$ sont identiques ou différents et représentent:

  un radical alkyle linéaire ou ramifié contenant de 1 à 4 atomes de carbone;
  les radicaux $R_7$ et $R_8$ pouvant former ensemble un radical alkylène contenant de 3 à 6 atomes de carbone;
  les radicaux $R_6$ et $R_7$ ou $R_6$ et $R_8$ pouvant former ensemble un radical alkylène, alcénylène ou alcadiénylène, contenant 4 atomes de carbone et constituant avec N un hétérocycle azoté;

- $R_9$ représente un radical alkyle linéaire ou ramifié contenant de 1 à 4 atomes de carbone ou un radical phényle;
- $R_{10}$ représente:

  un radical alkyle linéaire ou ramifié contenant de 1 à 4 atomes de carbone, semblable à ou différent de $R_9$;
  un radical alcényle linéaire ou ramifié, contenant de 2 à 12 atomes de carbone, de préférence de 4 à 8 atomes de carbone, et tout particulièrement un radical alcényle dérivé du diène conjugué à carbonyler;

- n représente un nombre entier supérieur ou égal à 1 et inférieur ou égal à 10 et de préférence inférieur ou égal à 6.
- Z représente du phosphore ou de l'arsenic;
- $R_{11}$, $R_{12}$ et $R_{13}$ sont identiques ou différents et représentent

  un radical alkyle linéaire ou ramifié contenant de 1 à 16 atomes de carbone;
  un radical aryle contenant de 6 à 10 atomes de carbone.

A titre d'exemple de cations onium quaternaire répondant à la formule I on peut citer les cations:

tétraméthylammonium,
triéthylméthylammonium,
tributylméthylammonium,

triméthyl(n-propyl)ammonium,
tétraéthylammonium,
tétrabutylammonium,
tétrapropylammonium,
tétrapentylammonium,
tétrahexylammonium,
tétraheptylammonium,
tétraoctylammonium,
tétradécylammonium,
dodécyltriméthylammonium,
méthyltrioctylammonium,
tétradodécylammonium,
tétraoctadécylammonium,
hexadécyltriméthylammonium,
butyltripropylammonium,
méthyltributylammonium,
pentyltributylammonium,
heptyltributylammonium,
méthyldiéthylpropylammonium,
éthyldiméthylpropylammonium,
benzyltributylammonium,
benzyltriéthylammonium,
phényltriméthylammonium,
benzyltriméthylammonium,
benzyldiméthylpropylammonium,
benzyldiméthyloctylammonium,
benzyldiméthyltétradécylammonium,
benzyldiméthylhexadécylammonium,
diméthyldiphénylammonium,
méthyltriphénylammonium,
butène-2 yltriéthylammonium,
N,N-diméthyl-tétraméthylènammonium,
N,N-diéthyl-tétraméthylènammonium,
tétraméthylphosphonium,
tétrabutylphosphonium,
éthyltriméthylphosphonium,
triméthylpentylphosphonium,
octyltriméthylphosphonium,
dodécyltriméthylphosphonium,
triméthylphénylphosphonium,
diéthyldiméthylphosphonium,
dicyclohexyldiméthylphosphonium,
diméthyldiphénylphosphonium,
cyclohexyltriméthylphosphonium,
triéthylméthylphosphonium,
méthyl-tri(isopropyl)phosphonium,
méthyl-tri(n-propyl)phosphonium,
méthyl-tri(n-butyl)phosphonium,
méthyl-tri(méthyl-2 propyl)phosphonium,
méthyltricyclohexylphosphonium,
méthyltriphénylphosphonium,
méthyltribenzylphosphonium,
méthyl-tri(méthyl-4 phényl)phosphonium,
méthyltrixylylphosphonium,
diéthylméthylphénylphosphonium,
dibenzylméthylphénylphosphonium,
éthyltriphénylphosphonium,
tétraéthylphosphonium,
éthyl-tri(n-propyl)phosphonium,
triéthylpentylphosphonium,
hexadécyltributylphosphonium,
éthyltriphénylphosphonium,
n-butyl-tri(n-propyl)phosphonium,
butyltriphénylphosphonium,
benzyltriphénylphosphonium,

(β-phényléthyl)diméthylphénylphosphonium,
tétraphénylphosphonium,
triphényl(méthyl-4 phényl)phosphonium,
tétrakis(hydroxyméthyl)phosphonium,
tétrakis(hydroxy-2 éthyl)phosphonium,
tétraphénylarsonium.

Parmi les cations répondant à la formule II, on peut citer les cations:

N-méthylpyridinium,
N-éthylpyridinium,
N-hexadécylpyridinium,
N-méthylpicolinium.

Parmi les cations répondant à la formule III, on peut citer les cations:

bis(butène-2 yldiméthylammonium)-1,3 propane,
bis(triméthylammonium)-1,2 éthane,
bis(triméthylammonium)-1,3 propane,
bis(triméthylammonium)-1,4 butane,
bis(triméthylammonium)-1,3 butane.

Comme cation répondant à la formule IV, on peut citer:

le bis(triphénylphosphino)iminium.

A titre d'exemples d'anion dudit sel d'onium quaternaire, on peut citer les ions:

$F^-$ $ClO_4^-$ $PF_6^-$ $BF_4^-$ $B\Phi_4^-$ $PO_4^{3-}$ $HPO_4^{2-}$ $H_2PO_4^-$ $CH_3SO_3^-$

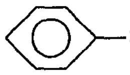—$SO_3$ $HSO_4^-$ $NO_3^-$ $SO_4^{2-}$ $Cl^-$ $Br^-$ $I^-$ $SCN^-$ $CN^-$ $RS^-$

où R représente un radical alkyle en $C_1$—$C_6$ ou aryle en $C_6$—$C_{10}$ éventuellement substitué.

Pour les raisons de commodité de mise en oeuvre, lesdits anions pourront être choisis parmi:

$PO_4^{3-}$ $HPO_4^{2-}$ $H_2PO_4^-$ $CH3SO_3^-$ 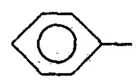—$SO_3^-$ $NO_3^-$ $SO_4^{2-}$ $PF_6^-$ $Cl^-$

$Br^-$ et de préférence $Cl^-$.

Parmi les alcools pouvant être présents pour réaliser l'opération de mise en contact, on peut citer les monoalcools aliphatiques linéaires ou ramifiés contenant de 1 à 4 atomes de carbone, et de préférence de 1 à 3 atomes de carbone pris seuls ou en mélange entre eux; l'alcool présent lors de ladite opération pourra favorablement être le même que celui mis en ouevre pour réaliser l'opération de carbonylation, et ce en raison de la quantité plus ou moins importante d'alcool non transformée généralement présente dans le milieu issu de l'opération de carbonylation.

Les solvants hydrocarbonés apolaires nécessaires à l'opération de mise en contact présentent une constante diélectrique $\varepsilon$ inférieure à 2,3 (valeur mesurée à 20—25°C) et tout particulièrement inférieure à 2,1.

Parmi ceux-ci on peut citer: le pentane ($\varepsilon$= 1,84), l'isopentane ($\varepsilon$= 1,84), l'hexane ($\varepsilon$= 1,89), le cyclohexane ($\varepsilon$= 2,02), l'octane ($\varepsilon$= 1,95), le cyclooctane ($\varepsilon$= 1,95), le triméthyl-2,-2,4 pentane (isooctane, $\varepsilon$= 1,94), le décane ($\varepsilon$= 1,99), le docécane ($\varepsilon$= 2,01), le tétradécane, l'hexadécane ou leurs mélanges du type éther de pétrole.

On dénommera ci-après par »réactifs de mise en contact«, le sel d'onium quaternaire, l'alcool et le solvant hydrocarboné apolaire présents lors de l'opération de mise en contact.

Les quantités desdits réactifs de mise en contact sont choisis de façon à obtenir une bonne séparation des deux phases après décantation, à aboutir au degré désiré de séparation du palladium et ce d'une manière économiquement intéressante.

La séparation entre la phase alcoolique et la phase organique est d'autant mieux réalisée que:

— le rapport molaire cation onium/palladium est élevé;
— le rapport pondéral sel d'onium quaternaire/alcool est élevé;
— la différence entre la constante diélectrique de l'alcool et celle du solvant hydrocarboné apolaire est grande.

Il est évident qu'économiquement il n'est pas intéressant d'utiliser le sel d'onium quaternaire en trop grand excés ni de mettre en oeuvre de trop grandes quantités d'alcool et de solvant.

Pour une bonne réalisation dudit procédé, pourront être présentes lors de l'opération de mise en contact:

- une quantité de sel d'onium quaternaire correspondant à un rapport molaire cation onium quaternaire/palladium d'au moins environ 10, et de préférence compris entre 20 et 200;
- et des quantités d'alcool et de solvant hydrocarboné apolaire au moins égales à celles nécessaires à la décantation du milieu de mise en contact en une phase alcoolique et en une phase organique.

Généralement, pourront être mises en oeuvre:

- une quantité d'alcool correspondant à un rapport pondéral sel d'onium quaternaire/alcool d'au moins environ 0,1, et de préférence comprise entre 0,25 et 30;
- une quantité de solvant correspondant à un rapport pondéral solvant/alcool supérieur à 1 environ et de préférence supérieur à 1,5 environ.

Un perfectionnement du procédé faisant l'objet de la présente invention, perfectionnement permettant une meilleure séparation de la phase alcoolique contenant le catalyseur au palladium, consiste à soumettre la phase alcoolique obtenue après décantation à une ou plusieurs opérations d'extraction et de séparation à l'aide du solvant hydrocarboné apolaire.

L'opération de mise en contact est réalisée dans des conditions de température permettant d'obtenir une bonne séparation des deux phases et ce d'une manière économiquement intéressante. La miscibilité relative entre un alcool et un alcane diminue généralement lorsque la température diminue; il a été constaté que la solubilité du sel d'onium quaternaire dans l'alcool diminue également mais que celle-ci demeure toutefois élevée; ainsi l'opération de mise en contact peut être réalisée à basse température et de préférence à une température voisine de la température ambiante.

La présente invention a également pour objet les différents modes de réalisation du procédé de séparation du palladium des produits issus de la carbonylation des diènes conjugués par de l'oxyde de carbone en présence d'un alcool, d'un hydracide halogéné et d'un catalyseur au palladium.

Un premier mode de réalisation dudit procédé est caractérisé:

- en ce que le milieu issu de l'opération de carbonylation contenant éventuellement de l'alcool non transformé, est additionné d'un sel d'onium quaternaire en quantité ci-dessus définie, d'un solvant hydrocarboné apolaire et éventuellement d'alcool, les quantités de solvant et d'alcool étant ajustées de façon à obtenir une séparation de la phase alcoolique et de la phase organique après décantation;
- et en ce que l'on récupère la phase alcoolique contenant l'alcool, le sel d'onium quaternaire et le catalyseur au palladium.

Un deuxième mode de réalisation dudit procédé est caractérisé en ce que:

- le sel d'onium quaternaire est choisi parmi ceux dont l'anion est une base »dure« ou »intermédiaire« (selon la définition classique donnée par R. PEARSON dans J. Chem. Ed, 45, 581–7, 1968).
- ledit sel d'onium quaternaire est mis en oeuvre en totalité ou en partie au cours de la réaction de carbonylation;
- le milieu issu de l'opération de carbonylation contenant le sel d'onium et éventuellement de l'alcool non réagi est additionné de sel d'onium si nécessaire de façon à respecter le rapport molaire onium/Pd ci-dessus défini, d'un solvant hydrocarboné apolaire et éventuellement d'alcool, les quantités de solvant et d'alcool étant ajustées de façon à obtenir une séparation de la phase alcoolique et de la phase organique après décantation;
- l'on récupère la phase alcoolique contenant l'alcool, le sel d'onium quaternaire et le catalyseur au palladium, phase alcoolique que l'on recycle éventuellement dans la réaction de carbonylation.

Parmi les bases »dures« ou »intermédiaires« pouvant constituer l'anion desdits sels d'onium, on peut citer à titre d'exemple les ions:

$F^-$ $ClO_4^-$ $PF_6^-$ $BF_4^-$ $B\Phi_4^-$ $PO_4^{3-}$ $HPO_4^{2-}$ $CH_3SO_3^-$

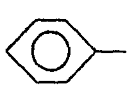 $-SO_3^-$ $HSO_4^-$ $NO_3^-$ $SO_4^{2-}$ $Cl^-$ $Br^-$ $H_2PO_4^-$.

Pour des raisons de commodité de mise en oeuvre, lesdits anions pourront être choisis parmi:

$PO_4^{3-}$ $HPO_4^{2-}$ $H_2PO_4^-$ $CH_3SO_3^-$ 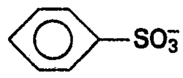 $NO_3^-$ $SO_4^{2-}$ $PF_6^-$ $Cl^-$

$Br^-$, et de préférence $Cl^-$.

Ce deuxième mode de réalisation est tout particulièrement intéressant, étant donné que la phase alcoolique contenant le sel d'onium quaternaire et le catalyseur au palladium, récupérée après décantation peut être directement recyclée dans le milieu soumis à la carbonylation sans modification importante des performances du catalyseur.

En effet, il a été constaté (ceci fait l'objet de la demande de brevet français n° 81.01205 déposée le 23 Janvier 1981 au nom de la Demanderesse), que les diènes conjugués peuvent être carbonylés en présence d'alcool, d'hydracide halogéné, d'un catalyseur au palladium et d'un sel d'onium quaternaire présentant un anion »dur« ou »intermédiaire«; un effet bénéfique, notamment sur le taux de conversion des diènes et sur la sélectivité en esters carboxyliques $\beta,\gamma$-insaturés recherchés, a été constaté pour une quantité de sel d'onium correspondant à un rapport molaire cation onium/palladium d'au moins 0,5 environ, en particulier lorsque ledit cation onium répond à l'une des formules I à IV ci-dessus.

Un effet particulièrement intéressant a été constaté pour un rapport molaire cation onium/palladium compris entre 1 et 15.

La présence dudit sel d'onium en une quantité beaucoup plus importante, correspondant à celle nécessaire à la séparation subséquente du palladium, n'étant absolument pas nocive pour l'opération de carbonylation, on voit immédiatement l'intérêt de ce deuxième mode de réalisation avec possibilité de recyclage de la phase alcoolique contenant le catalyseur au palladium et le sel d'onium.

Une variante des deux modes de réalisation consiste à mettre en oeuvre tout ou partie du solvant organique apolaire au cours de la réaction de carbonylation.

Un perfectionnement des deux modes de réalisation consiste à faire subir à la phase alcoolique séparée un traitement complémentaire consistant à soumettre ladite phase alcoolique à une ou plusieurs opérations d'extraction et de séparation à l'aide dudit solvant hydrocarboné apolaire.

Le procédé faisant l'objet de l'invention est tout particulièrement adapté à la séparation du palladium de produits issus de la réaction de carbonylation de diènes conjugués présentant dans leur molécule le squelette butadiène-1,3 et de monoalcools aliphatiques linéaires ou ramifiés contenant de 1 à 4 atomes de carbone, en présence d'acide chlorhydrique et d'un catalyseur au palladium, à une température comprise entre 50 et 150°C sous une pression de CO comprise, de préférence, entre 50 et 250 bars.

Parmi les diènes conjugués présentant dans leur molécule le squelette butadiène-1,3, on peut citer:

les diènes aliphatiques linéaires ou ramifiés, contenant de 4 à 12 atomes de carbone, et de préférence de 4 à 8 atomes de carbone, éventuellement substitués par des groupes inertes tels que: phényle, cyclohexyle, nitro, oxo et en particulier alcoxycarbonyle;
les diènes cycliques contenant de 6 à 8 atomes de carbone.

Comme exemples concrets de diène conjugué, on peut citer le butadiène-1,3, l'isoprène, le pipérylène, l'hexadiène-1,3, l'hexadiène-2,4, le chloroprène, le cyclohexyl-1 butadiène-1,3, le phényl-1 butadiène-1,3, l'octadiène-2,4, le méthyl-3 pentadiène-1,3 le méthyl-2 pentadiène-2,4, le cyclohexadiène-1,3, le cyclooctadiène-1,3 etc. ..., éventuellement substitués par un groupe alcoxycarbonyle tel que le pentadiène-2,4 oate de méthyle.

L'alcool pouvant être mis en oeuvre pour réaliser les opérations de carbonylation et de mise en contact est un monoalcool contenant de 1 à 4 atomes de carbone, de préférence de 1 à 3 atomes de carbone lorsque l'opération de carbonylation est réalisée en présence d'un sel d'onium quaternaire présentant un anion »dur« ou »intermédiaire«; ledit alcool peut contenir de 2 à 4 atomes de carbone et de préférence 2 ou 3 atomes de carbone lorsque l'opération de carbonylation est réalisée en l'absence de sel d'onium quaternaire.

L'opération de carbonylation est favorablement réalisée en présence d'acide chlorhydrique qui peut être introduit dans le milieu de carbonylation sous forme gazeuse ou sous forme d'un composé organique susceptible de libérer HCl dans le milieu, par exemple sous forme de chloro-1 butène-2 ou de chloro-3 butène-1 dans le cas de la carbonylation du butadiène.

Parmi les catalyseurs au palladium présents lors de l'opération de carbonylation, on peut citer:

le palladium métallique déposé sur un support, tel que le charbon, l'alumine, la silice ...
les oxydes de palladium
les sels ou complexes de palladium II dont l'anion coordiné au cation Pd est une base »dure« ou »intermédiaire«, notamment les sels ou les complexes $\pi$-allyliques du Pd dont l'anion coordiné au cation Pd est choisi parmi les anions suivants: carboxylates tels que formiate, acétate, propionate, benzoate; $SO_4^{2-}$; $NO_3^-$; acétyl-acétonate; halogénures tels que $Cl^-$ et $Br^-$, et de préférence $Cl^-$;
ou les complexes de palladium zéro comprenant des ligands organiques ne contenant pas d'éléments du groupe VB, complexes tels que le bis(dibenzalacétone) Pd ou le bis(cyclooctadiène-1,5)Pd.

Les quantités de réactifs à utiliser pour réaliser l'opération de carbonylation peuvent varier entre des limites très larges; il est évident que lesdites quantités seront choisies de manière à ce que le procédé soit économiquement intéressant.

Ainsi, bien qu'il soit possible d'utiliser de 0,5 à 10 fois la quantité d'alcool stoechiométriquement nécessaire, il est préférable afin d'avoir une conversion maximum du diène conjugué tout en évitant de trop diluer le milieu par de l'alcool, de réaliser le procédé avec un rapport molaire alcool/diène conjugué compris entre environ 0,8 et 5 et de préférence voisin de 1.

De même la bonne activité des catalyseurs au palladium permet l'emploi desdits catalyseurs en quantité très faible (correspondant à un rapport molaire diène conjugué/palladium de l'ordre de 2500); l'emploi d'une quantité élevée de catalyseur (correspondant à un rapport molaire diène conjugué/palladium de l'ordre de 100) n'est pas nuisible; le but recherché étant de réaliser une opération de carbonylation suffisamment rapide et sélective, sans dépenser trop de catalyseur, un rapport diène conjugué/palladium compris entre environ 100 et 2000 est généralement préférable.

La quantité d'hydracide halogéné à utiliser correspond à un rapport molaire hydracide halogéné/palladium supérieur à 2 environ. Toutefois, afin d'éviter tout risque de dégradation de l'alcool en chlorures d'alkyle et oxydes de dialkyle (dégradation due à une concentration trop importante du milieu en hydracide), un rapport molaire hydracide halogéné/palladium compris entre 2 et 150, et de préférence entre 5 et 100, pourra être favorablement choisi.

Les entités élémentaires correspondant aux termes »moles« sont les suivantes:

| | |
|---|---|
| alcool: | molécule-gramme; |
| diène conjugué: | molécule-gramme; |
| hydracide halogéné: | molécule-gramme; |
| palladium: | atome-gramme; |
| cation onium quaternaire: | ion-gramme. |

Les exemples suivants sont donnés à titre indicatif et ne peuvent être considérés comme une limite du domaine et de l'esprit de l'invention.

Les abréviations figurant dans les tableaux correspondant à ces exemples ont le sens suivant:

| | |
|---|---|
| Ex: | exemple; |
| BD: | butadiène; |
| ROH: | alcool; |
| Me: | méthyl; |
| Et: | éthyl; |
| Met: | méthoxy-2 éthyl; |
| Bu: | n-butyl; |
| $\Phi$: | phényl; |
| Pd(DBA)$_2$: | bis(dibenzalacétone)Pd; |
| [<(Pd-Cl]$_2$: | bis($\pi$-allyl palladium chlorure); |
| catalyseur: | catalyseur au palladium; |
| cocatalyseur: | HCl ou produit de la réaction HCl + diène conjugué mis en oeuvre; |
| onium: | sel d'onium quaternaire; |
| alcane: | solvant hydrocarboné apolaire; |
| MR: | masse réactionnelle issue de la carbonylation (contenant le butadiène non transformé ainsi que des produits volatils qui s'évaporent lors de l'opération ultérieure de mise en contact réalisée à température ambiante); |
| $P_3$: | ester pentène-3 oïque; |
| $P_4$: | ester pentène-4 oïque; |
| $P_2$: | ester pentène-2 oïque; |
| P': | ester méthyl-2 butène-3 oïque; |
| $C_9$: | ester nonadiène-3,8 oïque; |
| $C_6$: | diesters alcoyliques en $C_6$ (en majorité du méthyl-2 glutarate de dialkyle); |
| $HC_8$: | dimères de butadiène (essentiellement du vinyl-4 cyclohexène); |
| PA: | ester pentanoïque et ester méthyl-2 butanoïque; |
| $ROC_4$: | alcoxy-3 butène-1 et alcoxy-1 butène-2; |
| $ClC_4$: | chloro-3 butène-1 et chloro-2 butène-2; |
| ClPA: | ester chloropentanoïque; |
| Cl: | chlorure d'alkyle; oxyde de dialkyle; (réaction parasite de HCl sur l'alcool); |
| TT: | taux de conversion global du butadiène (en mole %); |
| RR: | taux de conversion partiel (en mole %) pour chaque produit obtenu par rapport au butadiène chargé, avec $TT = \Sigma_i RR_i$. |

Ne sont pris en compte pour le calcul de TT que les RR des produits suivants: $P_3$, $P_4$, $P_2$, P', $C_9$, $C_6$, $HC_8$, PA, $ROC_4$ et ClPA; en effet, les chlorobutènes ($ClC_4$) sont équivalents à un mélange butadiène + HCl carbonylable en $P_3$.

RT: sélectivité (en moles %) pour chaque produit avec $RT = \frac{RR}{TT}$

RRCl: taux de conversion partiel (en mole %) en chlorure d'éthyle et éther diéthylique, par rapport à l'éthanol chargé.

A: activité spécifique du catalyseur, exprimée en nombre de moles de $P_3$ et de $P_4$ obtenues par atg de Pd et par heure.

CO: CO gazeux technique contenant environ 0,8% en volume d'hydrogène qui n'intervient pas notablement pour la réaction de carbonylation.

## Exemple 1

### 1ère étape de carbonylation

Dans un autoclave de 125 $cm^3$ en alliage nickel-molybdène de marque HASTELLOY $B_2$, on introduit sous courant d'argon:

- 0,075 g (soit 0,423 mmole) de chlorure de palladium II anhydre;
- 30,8 g (soit 513 mmoles) de propanol-2
- 3,82 g (soit 42,25 mmoles) de chloro-1 butène-2, ce qui correspond à un rapport molaire HCl/Pd de 100.

L'autoclave est fermé; on y transverse 13 g (soit 240,7 mmoles) de butadiène, ce qui correspond à un rapport molaire butadiène/Pd de 569 et à un rapport molaire alcool/butadiène de 2,13.

L'autoclave agité par secousses est porté à 120°C avec alimentation de CO gazeux technique contenant environ 0,8% en volume d'hydrogène à pression totale constante de 145 bars.

On laisse la réaction se dérouler pendant 2 heures à cette température. L'autoclave est ensuite refroidi à 15°C et on dégaze lentement.

On récupère 46,67 g d'une solution homogène jaune contenant 43,6% en poids d'isopropanol et 42,3% en poids de pentènoates d'isopropyle.

On donne au tableau I les résultats de l'analyse chromatographique en phase gazeuse de la solution obtenue ainsi que les RT et RR correspondant à chaque produit formé.

Le taux de conversion global du butadiène TT est de 61,9 moles%.

L'activité spécifique A du catalyseur est de 166 $h^{-1}$.

### Etapes de mise en contact décantation et séparation

#### Premier Essai

On prend 1,21 g de la solution jaune obtenue, contenant 1,17 mg (soit 0,011 mmole) de Pd, auquel on ajoute 6,40 g de n-octane; la solution reste homogène.

On ajoute ensuite 0,21 g de $PBu_4^+Cl^-$, la composition ainsi obtenue contient:

- 6,7% en poids d'isopropanol,
- 6,7% en poids d'esters pentènoïques,
- 2,7% en poids de $PBu_4^+Cl^-$, ce qui correspond à un rapport molaire $PBu_4^+$/Pd de 65 et à un rapport pondéral $PBu_4^+Cl^-$/isopropanol de 0,40.
- 81,1% en poids d'octane, ce qui correspond à un rapport pondéral octane/alcool de 12,

Le milieu se sépare en deux phases:

- une phase supérieure incolore (7,03 g) contenant l'octane et

  environ 90% du pentène-3 oate d'isopropyle présent au début de l'opération de mise en contact;
  environ 100% du nonadiène-3,8 oate d'isopropyle et des diesters en $C_6$.
  et moins de 2 ppm de palladium.

- une phase inférieure jaune visqueuse (0,79 g) contenant l'isopropanol résiduel, le sel de phosphonium et plus de 99% du catalyseur au palladium traité.

2ème étape de carbonylation

Cette phase alcoolique est engagée dans une nouvelle étape de carbonylation, avec des charges identiques de réactifs et dans les mêmes conditions qu'à la première étape de carbonylation; le pentène-3 oate d'isopropyle produit correspond à une activité spécifique d'environ 210 $h^{-1}$.

Deuxième Essai

On réalise de la même façon une opération de mise en contact

- sur 1,37 g de solution jaune issue de l'opération de carbonylation et contenant 1,32 mg (soit 0,012 mmoles) de palladium;
- à l'aide de 2,24 g d'octane et de 0,55 g de $PBu_4^+Cl^-$.

La composition ainsi obtenue contient:

- 14,4% en poids d'isopropanol
- 14% en poids d'esters penténoïques
- 13,2% en poids de $PBu_4^+Cl^-$, ce qui correspond à un rapport molaire $PBu_4^+Cl^-/Pd$ de 150 et à un rapport pondéral $PBu_4^+Cl^-$/isopropanol de 0,92;
- 53,8% en poids d'octane, ce qui correspond à un rapport pondéral octane/alcool de 3,7.

Le milieu se sépare en deux phases:

- une phase supérieure incolore (2,94 g) contenant:

  environ 85% du pentène-3 oate d'isopropyle présent au début de l'opération de mise en contact;
  environ 100% du nonadiène-3,8 oate d'isopropyle et des diesters en $C_6$;
  et moins de 3 ppm de palladium.

- une phase inférieure jaune visqueuse (1,21 g) contenant l'isopropanol résiduel, le sel de phosphonium et plus de 99% de catalyseur au palladium traité.

Exemple 2

Etape de carbonylation

On réalise une opération de carbonylation dans des conditions voisines de celles de l'exemple 1, en présence:

- d'éthanol comme alcool
- de HCl gazeux comme cocatalyseur.

Les quantités de réactifs mises en oeuvre sont les suivantes:

- 10 g (soit 185,2 mmoles) de butadiène
- 36,8 g (soit 800 mmoles) d'éthanol absolu
- 60,3 mg (soit 0,34 mmoles) de $PdCl_2$
- 248,2 mg (soit 6,8 mmoles) d'HCl gazeux,
  ce qui correspond aux rapports molaires suivants: HCl/Pd = 20 et éthanol/butadiène = 4,3 et butadiène/Pd = 545.

L'opération de carbonylation est réalisée pendant 2 heures à 120°C et une pression totale constante de 145 bars avec alimentation continue d'oxyde de carbone.

On récupère après carbonylation 45,9 g de solution homogène de couleur jaune très claire. On donne au tableau II les résultats de l'analyse chromatographique en phase gazeuse de la solution obtenue ainsi que les RT et RR correspondant à chaque produit formé.

Le taux de conversion global TT du butadiène est de 24,3 moles%.

L'activité spécifique A du catalyseur est de 47 $h^{-1}$.

Etapes de mise en contact, décantation et séparation

A un échantillon de 2,83 g de masse réactionnelle contenant 2,18 g d'éthanol, on ajoute 5,68 g de n-décane, ce qui correspond à un rapport pondéral alcane/alcool de 2,6; le mélange reste homogène. La séparation en deux phases est provoquée par l'addition de 0,60 g de chlorure de tétra (hydroxyméthyl)-phosphonium, ce qui correspond à un rapport pondéral $P(CH_2OH)_4^+Cl^-$/alcool de 0,27 et à un rapport molaire onium/Pd de 150.

La phase supérieure incolore (8,03 g) est constituée essentiellement de n-décane et contient moins de 5 ppm de Pd.

La phase inférieure (1,05 g) d'une intense coloration verte contient l'éthanol résiduel et la quasi totalité du sel de phosphonium et du catalyseur au palladium.

Exemple 3

L'opération de carbonylation décrite à l'exemple 2 est réalisée dans des conditions voisines, pendant 2 heures à 120°C sous pression totale constante de 145 bars avec alimentation continue de CO. Les quantités de réactifs mises en oeuvre sont les suivantes:

- butadiène 23 g (soit 426 mmoles)
- éthanol absolu 66,2 g (soit 1440 mmoles)
- $PdCl_2$ 150 mg (soit 0,845 mmoles)
- HCl gazeux 1,54 g (soit 42,2 mmoles).

Les rapports molaires sont:

$$\frac{HCl}{Pd} = 50 \qquad \frac{BD}{Pd} = 504$$

On récupère après carbonylation 88,8 g de masse réactionnelle, solution homogène jaune clair. On donne au tableau III les résultats de l'analyse chromatographique et les valeurs de RT et RR.

Le taux de conversion du butadiène TT est de 53,9%, l'activité A du catalyseur est de 121 $h^{-1}$.

La composition pondérale de la masse réactionnelle est de 30% en P 3, de 62% en éthanol. On y ajoute 50 g de n-octane et on la soumet à une distillation, qui en extrait 66,5 g d'un mélange contenant 20,3 g d'octane et 41,2 g d'éthanol. On ajoute à la phase non volatile 9 g de $PBu_4^+Cl^-$; la composition est alors de:

- 14,1 g en éthanol
- 28,8 g en octane
- 9,0 g en $PBu_4^+Cl^-$

soit un rapport pondéral alcane/alcool de 2,04, un rapport pondéral onium/alcool de 0,64, un rapport molaire onium/Pd de 36.

Il y a séparation entre deux phases:

- une phase supérieure organique, incolore de 55,7 g contenant 3,5 ppm de Pd
- une phase inférieure alcoolique, de couleur verte-jaune, de 23,5 g.

Exemples 4 A 8

Etape de carbonylation

Des opérations de carbonylation sont réalisées dans des conditions voisines de celles de l'exemple 1.

- en présence d'éthanol comme alcool au lieu de l'isopropanol
- et en outre en présence d'un solvant hydrocarboné apolaire choisi parmi le n-octane, le n-tétradécane et le n-pentane.

Lesdites opérations sont réalisées à 120°C pendant 2 heures à une pression totale constante de 120 bars avec alimentation continue de CO.

Les quantités de réactifs mises en oeuvre et les résultats de l'opération de carbonylation figurent aux tableaux IV A et IV B.

Etapes de mise en contact, décantation et séparation

On soumet divers échantillons des solutions obtenues en utilisant les différents solvants apolaires à des opérations de mise en contact à l'aide des différents sels d'onium figurant au tableau IV C.
Ce tableau indique également:

- la composition de l'échantillon à traiter
- les quantités de sel d'onium mises en oeuvre
- la quantité de sel d'onium minimum à partir de laquelle on a constaté une séparation de phases (»onium minimum«)
- la couleur et la composition de la phase organique supérieure,
- la couleur et la composition de la phase alcoolique inférieure.

L'exemple 4 a été répété en remplaçant $NBu_4^+BF_4^-$, par $P\Phi_4^+Cl^-$, $NBu_4^+CH_3SO_3^-$, $\Phi_3P = N = P\Phi_3^+Cl^-$; on constate de la même façon une séparation en deux phases, une phase organique supérieure incolore, une phase alcoolique inférieure colorée à savoir:

- jaune paille en présence de $P\Phi_4^+Cl^-$,
- jaune très pâle en présence de $NBu_4^+CH_3SO_3^-$,
- jaune vif en présence de $\Phi_3P = N = P\Phi_3^+Cl^-$.

Exemple 9

9 a) Etape de carbonylation

On réalise une opération de carbonylation dans des conditions voisines de celles de l'exemple 1 en présence des quantités de réactifs figurant au tableau VA.
Ladite opération est réalisée pendant 3 heures à 100°C à une pression totale constante de 100 bars avec alimentation continue de CO.
Les résultats de ladite opération figurent au tableau VB.

9 b) Etapes de mise en contact, décantation et séparation

La masse réactionnelle obtenue est ensuite additionnée de 70 g de n-octane.
Le milieu se sépare en deux phases (dont les poids sont mentionnés au tableau VC):

- une phase supérieure incolore dont la composition est donnée au tableau VD et contenant au plus 4 ppm de Pd.
- une phase inférieure jaune vert contenant l'éthanol et au moins 99,3% du Pd.

9 c) Recyclage

La phase alcoolique contenant environ 0,421 matg de Pd est recyclée dans l'autoclave de carbonylation.
On introduit en outre dans ledit autoclave, de l'éthanol, du chloro-1 butène-2 et du butadiène selon des quantités correspondant aux rapports molaires indiqués à l'étape 9 a).
L'opération de carbonylation est réalisée pendant 3 heures à 100°C à une pression totale constante de 100 bars avec alimentation continue de CO.
Les résultats de l'opération de carbonylation figurent au tableau VB.

Exemples 10—23

L'opération de carbonylation décrite à l'exemple 1 est réalisée en présence:

- de différents alcools choisis parmi: le méthanol (MeOH), l'éthanol (EtOH), le méthoxy-2 éthanol (MetOH).
- de différents catalyseurs au palladium: $PdCl_2$, le bis(dibenzalacétone)Pd, le bis($\pi$-allylpalladium chlorure);
- d'HCl gazeux ou de chlorobutènes
- et en outre de différents sels d'onium quaternaires présentant un anion »dur« ou »intermédiaire«.

La nature, les quantités de réactifs et les conditions de température et de pression de CO, utilisées, sont indiquées aux tableaux VA et VIA.

Les résultats des opérations de carbonylation figurent aux tableaux VB et VIB.

Les solutions obtenues ou des échantillons de solutions sont ensuite additionnés d'un alcane apolaire selon des quantités indiquées aux tableaux VC et VIC.

L'analyse des phases alcooliques et organiques obtenues après décantation est indiquée au tableau VD.

Les phase alcooliques séparées peuvent ensuite être éventuellement soumises à un ou plusieurs traitements complémentaires d'extraction-séparation à l'aide d'un alcane apolaire selon des quantités indiquées aux tableaux VC et VIC. Figure au tableau VD l'analyse des différentes phases organiques successives éventuelles.

L'éther de pétrole mise en oeuvre à l'exemple 17 est une coupe de distillation de point d'ébullition compris 40 et 65°C et constituée d'alcanes contenant essentiellement 5 atomes de carbone.

### Essai 19'''

Le milieu réactionnel issu de l'opération de carbonylation décrite à l'exemple 19 est soumis à une opération de mise en contact à l'aide de solvants de constante diélectrique supérieure à 2,3 au lieu de solvants apolaires. Aucune séparation n'a pu être constatée en mettant en oeuvre pour 5 g de masse réactionnelle l'un des solvants apolaires suivants:

- toluène (de 0 à 7,55 g) $\varepsilon = 2{,}38$
- chloroforme (de 0 à 11,8 g) $\varepsilon = 4{,}81$

### Exemple 24

On réalise une opération de carbonylation semblable à celle de l'exemple 12, le milieu à carbonyler contenant en outre de l'octane.

Les quantités de réactifs sont les suivantes:

- 269 mmoles de butadiène,
- 261 mmoles d'éthanol,
- 0,423 mmoles de $PdCl_2$ (rapport molaire butadiène/Pd = 635)
- 8,45 mmoles de chloro-1 butène-2 (rapport molaire HCl/Pd = 20)
- 42,3 mmoles de $PBu_4^+Cl^-$ (rapport molaire onium/Pd = 100).
- 14 g d'octane.

L'opération est réalisée pendant 2 heures à 120°C sous une pression totale constante de 120 bars.

On récupère 56,52 g d'une solution jaune contenant 3,30 g d'éthanol et 14 g d'octane.

Les résultats de l'analyse chromatographique en phase gazeuse sont donnés au tableau VIB ainsi que les RR et RT pour chaque produit.

Le taux de conversion global du butadiène TT est de 62,7%, l'activité du catalyseur A est de 181 $h^{-1}$

La solution est ensuite additionnée de 100 $cm^3$ d'octane.

On a ainsi en présence:

- 45 mg de palladium,
- 3,3 g d'éthanol
- 84 g d'octane
- 12,46 g de $PBu_4^+Cl^-$, ce qui correspond à un rapport molaire $PBu_4^+Cl^-$/Pd de 100 environ, à un rapport pondéral $PBu_4^+Cl^-$/éthanol de 3,8, et à un rapport pondéral octane/éthanol de 25,4.

On récupère après décantation:

- 23,6 g d'une phase alcoolique jaune orange,
- et 98,4 g d'une phase organique incolore contenant moins de 2,5 ppm de palladium.

### Exemple 25

On réalise une opération de carbonylation en mettant en oeuvre les quantités de réactifs suivantes:

- pentadiène-2,4 oate de méthyle 4,825 g (soit 43 mmoles)
- méthanol 1,4 g (soit 43,8 mmoles)

| | |
|---|---|
| – chloro-1 butène-2 | 385 mg (soit 4,25 mmoles) |
| – $PdCl_2$ | 15 mg (soit 0,085 mmole) |
| – $PBu_4^+Cl^-$ | 2,51 g (soit 8,5 mmoles). |

L'opération est réalisée pendant 2 heures à 90°C sous une pression de 145 bars. On récupère 9,06 g d'une solution homogène assez visqueuse, de couleur jaune citron.

Les résultats de l'analyse chromatographique en phase gazeuse de la solution obtenue sont les suivants:

– 2,15 g de produits de carbonylation (diesters insaturés en $C_6$) constitués de:

37,7% de 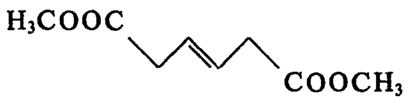

41,9% de 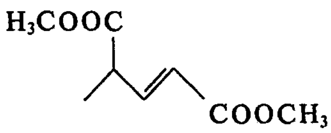

14,4% de 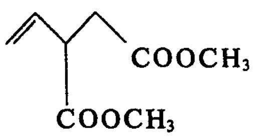

6,0% de 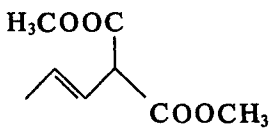

– et 1,62 g de dimères du pentadiène-2,4 oate de méthyle.

L'activité spécifique, calculée sur tous les diesters insaturés en $C_6$, est de 74 $h^{-1}$.

On prend 4,60 g de la masse réactionnelle, que l'on traite par 17 g d'octane. La composition est alors de:

– 0,46 g de méthanol
– 4,57 mg de Pd
– 1,27 g de $PBu_4Cl$
– 17 g d'octane

soit un rapport molaire onium/Pd = 100, des rapports pondéraux onium/alcool = 2,76, alcane/alcool = 37.

Il y a séparation entre deux phases:

– une phase supérieure incolore de 18,7 g contenant 26% des diesters insaturés en $C_6$, 63% des diesters en $C_{10}$ (»dimères«) 45% du substrat non réagi, moins de 1 ppm de palladium et moins de 5 ppm de phosphore.
– une phase inférieure jaune citron, visqueuse de 2,80 g.

## Exemple 26

On réalise une opération de carbonylation en mettant en oeuvre les quantités de réactifs suivantes:

| | |
|---|---|
| – isoprène | 27,25 g (soit 400 mmoles) |
| – éthanol | 18,40 g (soit 400 mmoles) |
| – chloro-1 butène-2 | 2,716 g (soit 30 mmoles) |
| – bis($\pi$-allyl-palladium-chlorure) | 179,7 mg (soit 1,0 matg de Pd) |
| – $PBu_4Cl$ | 8,85 g (soit 30 mmoles) |

ce qui correspond aux rapports molaires suivants:

$$\frac{\text{diène}}{\text{Pd}} = 400 \qquad \frac{\text{HCl}}{\text{Pd}} = 30$$

L'opération est réalisée pendant 4 h à 100°C sous une pression totale de 200 bars. On récupère 62,1 g d'une solution homogène, de couleur jaune citron.

L'analyse chromatographique indique que l'on obtient 33,75 g de produits de carbonylation (esters insaturés en $C_6$) constitués de 94% de méthyl-4 pentène-3 oate d'éthyle.

L'activité spécifique, calculée sur le méthyl-4 pentène-3 oate d'éthyle, est de 56 $h^{-1}$.

On prend 31 g de la masse réactionnelle, que l'on traite par 50 $cm^3$ de n-octane. La composition est alors de:

- 53,2 mg de palladium
- 4,42 g de $PBu_4Cl$
- 2,92 g d'éthanol
- 35 g d'octane

ce qui correspond à un rapport molaire onium/Pd = 30, et à des rapports pondéraux onium/alcool = 1,52 et alcane/alcool = 12.

Après séparation entre les deux phases, on traite à nouveau la phase inférieure colorée par 2 fois 25 $cm^3$ d'octane. Après décantation, on rassemble toutes les phases supérieures incolores, et on récupère ainsi:

- une phase inférieure éthanolique orange, homogène, limpide, de 8,70 g qui contient 98,6% du Pd;
- une phase supérieure octanique, incolore, limpide, d'un volume de 126 $cm^3$ (91,6 g); l'octane a donc extrait 26 $cm^3$ (21,6 g) de produits organiques (soit 93% desdits produits); cette phase contient environ 6 ppm de palladium.

Tableau I

| Produits formés | Poids g | mmoles | RT % | RR % |
|---|---|---|---|---|
| isopropoxybutène $ROC_4$ | 0,056 | 0,5 | 0,3 | 0,2 |
| vinyl-4 cyclohexène $HC_8$ | 0,141 | 2,6 | 1,7 | 1,1 |
| méthyl-2 butène-3 oate d'isopropyle P' | 0,375 | 2,6 | 1,72 | 1,1 |
| pentène-4 oate d'isopropyle $P_4$ | 0,064 | 0,5 | 0,3 | 0,2 |
| pentène-3 oate d'isopropyle $P_3$ | 19,727 | 138,9 | 93,2 | 57,7 |
| pentène-2 oate d'isopropyle $P_2$ | 0,074 | 0,5 | 0,3 | 0,2 |
| chloropentanoate d'isopropyle ClPA | 0,136 | 0,8 | 0,5 | 0,3 |
| nonadiène-3,8 oate d'isopropyle $C_9$ | 0,176 | 1,8 | 1,2 | 0,7 |
| diesters en $C_6$ | 0,182 | 0,8 | 0,5 | 0,3 |

Tableau II

| Produits formés | poids g | mmoles | RT % | RR % |
|---|---|---|---|---|
| éthoxybutène $ROC_4$ | 0,14 | 1,4 | 3,1 | 0,7 |
| vinyl-4 cyclohexène $HC_8$ | 0,12 | 2,2 | 4,9 | 1,2 |
| méthyl-2 butène-3 oate d'éthyle P' | 0,08 | 0,6 | 1,3 | 0,3 |

Suite

| Produits formés | poids g | mmoles | RT % | RR % |
|---|---|---|---|---|
| pentènes oate d'éthyle $P_3 + P_4$ | 4,13 | 32,2 | 71,7 | 17,4 |
| nonadiène-3,8 oate d'éthyle $C_9$ | 0,46 | 8,5 | 18,9 | 4,6 |
| diesters en $C_6$ | $\varepsilon$ | $\varepsilon$ | $\varepsilon$ | $\varepsilon$ |

Tableau III

| Produits formés | Poids g | mmoles | RT % | RR % |
|---|---|---|---|---|
| éthoxybutène $ROC_4$ | 0,40 | 4,0 | 1,8 | 0,9 |
| vinyl-4 cyclohexène $HC_8$ | 0,297 | 5,5 | 2,4 | 1,3 |
| méthyl-2 butène-3 oate d'éthyle P' | 0,845 | 6,6 | 2,9 | 1,6 |
| pentènes oate d'éthyle $P_3 + P_4$ | 26,26 | 205,1 | 90,0 | 48,5 |
| chloropentanoate d'éthyle ClPA | 0,131 | 0,8 | 0,3 | 0,1 |
| nonadiène-3,8 oate d'éthyle $C_9$ | 0,255 | 2,8 | 1,2 | 0,7 |
| diesters en $C_6$ | 0,485 | 2,4 | 1,0 | 0,6 |

Tableau IV A

| EX | BD mM | EtOH mM | Catalyseur mM $PdCl_2$ | Cocatalyseur mM | BD/Pd | HCl/Pd | alcane | MR poids g | MR aspect |
|---|---|---|---|---|---|---|---|---|---|
| 4 | 259 | 261 | 0,18 | 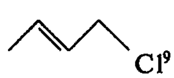 9 | 1440 | 50 | octane: 20 g | 41,0 | jaune clair |
| 5 | 259 | 261 | 0,423 | HCl 8,45 | 614 | 20 | octane: 20 g | 49,0 | jaune citron |
| 6 | 259 | 261 | 0,423 | HCl 21,1 | 614 | 50 | octane: 20 g | 50,7 | jaune orangé |
| 7 | 259 | 261 | 0,18 | 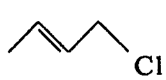 9 | 1440 | 50 | octane: 20 g | 41,0 | jaune clair |
| 8 | 268 | 261 | 0,423 | 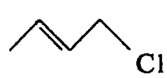 2,11 | 636 | 5 | tétradécane: 20 g | 46,5 | jaune |

Tableau IV B

| Ex | TT(%) | A($h^{-1}$) | P 3 + 4 RT% | P 3 + 4 RR% | P' RT% | P' RR% | C 9 RT% | C 9 RR% | HC 8 RT% | HC 8 RR% | ROC 4 RT% | ROC 4 RR% | RRCl | ROH résiduel |
|---|---|---|---|---|---|---|---|---|---|---|---|---|---|---|
| 4 | 14,0 | 84 | 83,7 | 11,7 | 1,4 | 0,2 | 3,3 | 0,5 | 9,1 | 1,3 | 2,5 | 0,3 | 1,4 | 10,3 g |
| 5 | 71,2 | 206 | 94,5 | 67,3 | 1,8 | 1,3 | 1,7 | 1,2 | 1,6 | 1,2 | 0,4 | 0,3 | 0,3 | 3,9 g |
| 6 | 71,8 | 210 | 95,4 | 68,5 | 2,2 | 1,9 | 0,5 | 0,4 | 1,2 | 0,8 | 0,7 | 0,5 | 1,2 | 5,0 g |
| 7 | 14,0 | 84 | 83,7 | 11,7 | 1,4 | 0,2 | 3,3 | 0,5 | 9,1 | 1,3 | 2,5 | 0,3 | 1,4 | 10,3 g |
| 8 | 32,3 | 80 | 77,6 | 25,1 | 1,5 | 0,5 | 14,1 | 4,6 | 6,0 | 1,9 | 0,8 | 0,3 | | 10,2 g |

Tableau IV C

| Exemples | 4 | 5 | 6 | 7 | 8 |
|---|---|---|---|---|---|
| Echantillon à traiter | | | | | |
| poids (g) | 41,0 | 49,0 | 50,7 | 41,0 | 46,5 |
| dont: | | | | | |
| éthanol (g) | 10,3 | 3,9 | 5,0 | 10,3 | 10,2 |
| P 3 (g) | 3,9 | 22,3 | 22,7 | 3,9 | 8,6 |
| alcane (g) | octane: 20 | octane: 20 | octane: 20 | octane: 20 | tétra-décane: 20 |
| soit rapports: | | | | | |
| Alcane/éthanol (pondéral) | 1,9 | 5,1 | 4,0 | 1,9 | 2,0 |
| Mise en contact | | | | | |
| sel d'onium | $NBu_4^+BF^-$ | $NBu_4^+Br^-$ | $PBu_4^+Cl^-$ | $NBu_4^+SCN^-$ | $PBu_4^+Cl^-$ |
| poids d'onium (g) | 2,62 | 2,75 | 5,37 | 5,17 | 4,75 |
| onium/alcool (pondéral) | 0,25 | 0,62 | 1,07 | 0,50 | 0,47 |
| onium/Pd (molaire) | 44 | 20 | 43 | 96 | 38 |
| minimum d'onium (g) | 0,41 | 1,15 | 1,72 | 3,28 | 2,14 |
| Phase alcoolique | | | | | |
| couleur | orangée | rouge | orangée | rouge vif | orangée |
| poids (g) | 17,2 | 16,5 | 27,6 | 20,6 | 19,9 |

Suite

| Exemples | 4 | 5 | 6 | 7 | 8 |
|---|---|---|---|---|---|
| Phase organique | | | | | |
| couleur | jaunâtre | jaunâtre | incolore | jaunâtre | incolore |
| poids (g) | 26,4 | 35,0 | 28,2 | 25,2 | 30,4 |
| P 3 (g) | 6,1 | 18,7 | 14,3 | 3,2 | |
| Pd | 53 ppm | 9 ppm | 47 ppm | 2 ppm | 22 ppm |

Tableau V A

| EX | BD | ROH | catalyseur | cocatalyseur | BD/PD | HCl/Pd | onium | onium | T°C | durée | PCO | MR | |
|---|---|---|---|---|---|---|---|---|---|---|---|---|---|
| | mM | mM | mM | mM | | | mM | Pd | | h | bars | poids g | couleur |
| 9 a | 269 | EtOH 261 | Pd $Cl_2$ 0,423 | 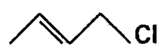 21,1 | 635 | 50 | $PBu_4^+Cl^-$ 42,3 | 100 | 100 | 3 | 145 | 39,71 | vert clair |
| 10 | 269 | MeOH 250 | 0,423 | 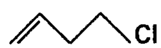 42,2 | 592 | 100 | $PBu_4^+Cl^-$ 42,3 | 100 | 80 | 2 | 120 | 25,65 | vert |
| 11 | 269 | EtOH 261 | 0,423 | 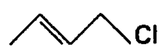 21,1 | 635 | 50 | $PBu_4^+Cl^-$ 42,3 | 100 | 120 | 2 | 145 | 43,5 | vert |
| 12 | 250 | EtOH 261 | 0,845 | 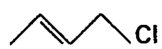 8,45 | 296 | 10 | $PBu_4^+Cl^-$ 42,3 | 50 | 120 | 2 | 120 | 42 | vert |

Suite

| EX | BD mM | ROH mM | catalyseur mM | cocatalyseur mM | BD/PD | HCl/Pd | onium mM | onium Pd | T°C | durée h | Pco bars | MR poids g | couleur |
|---|---|---|---|---|---|---|---|---|---|---|---|---|---|
| 13 | 296 | MetOH 261 | 0,423 | 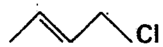 21,1 | 700 | 50 | $PBu_4^+Cl^-$ 42,3 | 100 | 120 | 2 | 145 | 42,79 | vert clair |
| 14 | 222 | EtOH 261 | 0,845 | 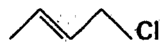 21,1 | 263 | 50 | $PBu_4^+Cl^-$ 42,3 | 50 | 120 | 4 | 145 | 44,85 | vert foncé |
| 15 | 259 | MeOH 250 | 1,27 | 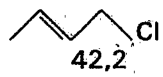 42,2 | 205 | 33 | $PBu_4^+Cl^-$ 42,3 | 33 | 90 | 2 | 120 | 33 | vert clair |
| 16 | 222 | EtOH 261 | 0,423 | 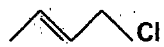 21,1 | 526 | 50 | $NBu_4^+H_2PO_4^-$ 16,9 | 40 | 120 | 2 | 90 | 31,1 | jaune clair |
| 17 | 232 | EtOH 261 | 0,306 | 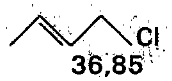 36,85 | 756 | 120 | $NBu_4^+CH_3SO_3^-$ 12,27 | 40 | 120 | 2 | 120 | 25,5 | jaune clair |

Tableau VI A

| EX | BD mM | ROH mM | catalyseur mM | cocatalyseur mM | BD/Pd | HCl/Pd | onium mM | onium Pd | T°C | durée h | PCO bars | MR poids g | couleur |
|---|---|---|---|---|---|---|---|---|---|---|---|---|---|
| 19 | 250 | EtOH 261 | $Pd(DBA)_2$ 0,423 | 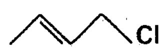 21,1 | 591 | 50 | $PBu_4^+Cl^-$ 42,3 | 100 | 120 | 2 | 200 | 43,1 | vert clair |

Suite

| EX | BD mM | ROH mM | catalyseur mM | cocatalyseur mM | BD/Pd | HCl/Pd | onium mM | onium Pd | T°C | durée h | PCO bars | MR poids g | couleur |
|---|---|---|---|---|---|---|---|---|---|---|---|---|---|
| 20 | 232 | EtOH 261 | 1,058 | 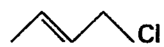 52,8 | 219 | 50 | $PBu_4^+Cl^-$ 42,3 | 40 | 120 | 1 | 145 | 41,3 | jaune vert |
| 21 | 333 | EtOH 261 | 0,423 | 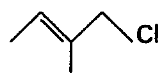 21,1 | 788 | 50 | $\Phi_3P{=}N{=}P\Phi_3^+Cl^-$ 10,6 | 25 | 140 | 1 | 145 | 43,2 | jaune vert |
| 22 | 204 | EtOH 261 | 0,423 | 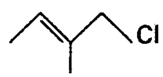 21,1 | 482 | 50 | $PBu_4^+Cl^-$ 42,3 | 100 | 120 | 2 | 145 | 40,72 | vert |
| 23 | 241 | EtOH 261 | 0,423 | 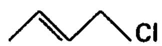 21,1 | 569 | 50 | $P\Phi_4^+Cl^-$ 35,1 | 83 | 120 | 2 | 145 | 37,7 | orange |
| 18 | 269 | EtOH 261 | [⟨(–PdCl]$_2$ 0,211 | 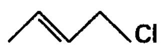 2,11 | 635 | 5 | $PBu_4^+Cl^-$ 42,2 | 100 | 120 | 2 | 200 | 41,1 | jaune |

Tableau V B

| EX | TT | A | p3 + 4 | | P' | | C9 | | HC8 | | ROC4 | | C6 | | RR | ROH résiduel en g | onium/ ROH |
|---|---|---|---|---|---|---|---|---|---|---|---|---|---|---|---|---|---|
| | % | h – 1 | RT% | RR% | RT% | RR% | RT% | RR% | RT% | RR% | RT% | RR% | RT% | RR% | Cl% | | |
| 9a | 35 | 69 | 92,6 | 32,4 | 1,7 | 0,6 | 1,7 | 0,6 | 2 | 0,7 | ε | ε | 1,1 | 0,4 | 1,7 | 7,33 | 1,70 |
| 9c | 32 | 63 | 92,3 | 29,6 | 1,6 | 0,5 | 1,8 | 0,6 | 1,9 | 0,6 | ε | ε | 1,3 | 0,4 | | | |

Suite

| EX | TT | A | P 3 + 4 | | P' | | C 9 | | HC 8 | | ROC 4 | | C 6 | | RR | ROH résiduel en g | onium/ ROH |
|---|---|---|---|---|---|---|---|---|---|---|---|---|---|---|---|---|---|
| | % | h − 1 | RT% | RR% | RT% | RR% | RT% | RR% | RT% | RR% | RT% | RR% | RT% | RR% | Cl% | | |
| 10 | 19 | 54 | 95,6 | 18,2 | 1,5 | 0,3 | 0,4 | 0,1 | 1,9 | 0,4 | ε | ε | 0,6 | 0,1 | 2 | 5,62 | 2,22 |
| 11 | 63 | 181 | 90,7 | 57,1 | 1,6 | 1,2 | 1,8 | 1,1 | 2 | 1,3 | ε | ε | 2,5 | 1,6 | 3,2 | 3,3 | 3,78 |
| 12 | 73 | 100 | 92,2 | 67,4 | 1 | 0,8 | 1,8 | 1,3 | 2 | 1,4 | ε | ε | 3,1 | 2,2 | 2,5 | 1,91 | 6,52 |
| 13 | 40 | 91 | 93 | 37,1 | 1,7 | 0,7 | 5 | 2 | ε | ε | ε | ε | 0 | 0 | 2,7 | 5,65 | 2,21 |
| 14 | 89,3 | 51 | 87,8 | 78,4 | 2,1 | 1,8 | 1,2 | 1,1 | 0,9 | 0,8 | ε | ε | 4,5 | 4 | 6 | 0,47 | 26,5 |
| 15 | 45,5 | 44 | 93,9 | 42,7 | 1,5 | 0,7 | 0,9 | 0,4 | 2,7 | 1,2 | ε | ε | 0,6 | 0,2 | ε | 5,07 | 2,46 |
| 16 | 49,4 | 111 | 85,9 | 42,4 | 1,9 | 0,9 | 7,8 | 3,8 | 2,5 | 1,2 | ε | ε | 1,6 | 0,8 | 1,1 | 8,19 | 0,70 |
| 17 | 28 | 94 | 88,7 | 24,8 | 2 | 0,6 | 1,7 | 0,5 | 4,6 | 1,3 | ε | ε | 1,1 | 0,3 | 4,5 | 7,55 | 0,76 |

Tableau VI B

| EX | TT | A | p 3 + 4 | | P' | | C 9 | | HC 8 | | ROC 4 | | C 6 | | RR | ROH résiduel en g | onium/ ROH résiduel en poids |
|---|---|---|---|---|---|---|---|---|---|---|---|---|---|---|---|---|---|
| | % | h − 1 | RT% | RR% | RT% | RR% | RT% | RR% | RT% | RR% | RT% | RR% | RT% | RR% | Cl% | | |
| 18 | 80,7 | 233 | 90,8 | 73,3 | 1,9 | 1,5 | 1,4 | 1,1 | 0,7 | 0,6 | ε | ε | 3,6 | 2,9 | 1,3 | 2,15 | 5,80 |
| 19 | 75 | 202 | 91,4 | 68,6 | 1,5 | 1,2 | 1,2 | 0,9 | 1 | 0,8 | – | – | 3,4 | 2,6 | 3,1 | 2,29 | 5,44 |

Suite

| EX | TT | A | p3 + 4 | | P' | | C9 | | HC8 | | ROC4 | | C6 | | RR | ROH résiduel en g | onium/ ROH résiduel en poids |
|---|---|---|---|---|---|---|---|---|---|---|---|---|---|---|---|---|---|
| | % | h − 1 | RT% | RR% | RT% | RR% | RT% | RR% | RT% | RR% | RT% | RR% | RT% | RR% | Cl% | | |
| 20 | 35,6 | 142 | 91,4 | 32,5 | 1,7 | 0,6 | 0,7 | 0,3 | 2,6 | 0,9 | – | – | 1,5 | 0,5 | 5,9 | 6,38 | 1,95 |
| 21 | 56,8 | 390 | 87,8 | 49,9 | 1,6 | 0,9 | 1,9 | 1,1 | 1,7 | 1 | – | – | 2,8 | 1,6 | 1,7 | 5,73 | 2,12 |
| 22 | 81,4 | 170 | 87 | 70,8 | 1,3 | 1 | 1,3 | 1 | ε | ε | – | – | 6,9 | 5,6 | 3,2 | 2,75 | 4,53 |
| 23 | 65,1 | 165 | 89,1 | 58,0 | 1,4 | 0,9 | 2,2 | 1,4 | 0,4 | 0,3 | ε | ε | 3,6 | 2,3 | 2,1 | 32,55*) | 0,41 |
| 24 | 62,7 | 181 | 90,9 | 57 | 1,9 | 1,2 | 3,2 | 2 | 1,8 | 1,2 | 0,2 | 0,1 | 1,9 | 1,2 | 1,1 | 3,3 | 3,78 |

*) addition supplémentaire d'alcool jusqu'à 32,55 g pour solubiliser tout le sel d'onium.

Tableau V C

| EX | Addition d'alcane | alcane ROH pondéral | phase*) alcoolique | phase organique n°1 | traitements complémentaires | | |
|---|---|---|---|---|---|---|---|
| | | | | | alcane | phases organiques | |
| | | | g | g | | n° | poids g |
| 9 a | octane 70 g | 4,8 | 25,2 jaune-vert | 79,1 incolore | – | – | – |
| 10 | octane 35 g | 6,2 | 21,1 vert | 32,9 incolore | octane 35 g | n°2 | 35,2 |

Suite

| EX | Addition d'alcane | alcane ROH pondéral | phase*) alcoolique | phase organique n° 1 | traitements complémentaires | | |
|---|---|---|---|---|---|---|---|
| | | | | | alcane | phases organiques | |
| | | | g | g | | n° | poids g |
| 11 | octane 35 g | 10,6 | 16,5 vert | 42,4 incolore | octane 2 × 35 g | n°2<br>n°3 | 45,3<br>38,1 |
| 12 | octane 70 g | 36,6 | 16,3 vert | 95,9 incolore | – | – | – |
| 13 | octane 62 g | 11,0 | 19,8 vert | 81,1 incolore | – | – | – |
| 14 | isooctane 50 g | 106 | 20,0 orange | 81,1 incolore | – | – | – |
| 15 | octane 17,5 g | 3,4 | 17,3 brun clair | 20,8 incolore | octane 2 × 35 g | n°2<br>n°3 | 39,4<br>39,5 |

*) après tous les traitements éventuels à l'alcane.

Tableau VI C

| EX | Addition d'alcane | | alcane ROH pondéral | phase alcoolique g | | phase organique g | |
|---|---|---|---|---|---|---|---|
| 16 | éther de pétrole | 33 g | 3,7 | 14,6 | orange | 44,9 | incolore |
| 17 | octane | 35 g | 4,6 | 17,8 | orange | 40,7 | incolore |
| 18 | octane | 74 g | 34,6 | 20,2 | jaune | 94,1 | incolore |
| 19 | pentane pour 5 g de MR | 6,25 g | 23,5 | 2,7 | orange | 8,5 | incolore |
| 19' | cyclohexane pour 5 g de MR | 7,1 g | 26,7 | 2,9 | orange | 9,1 | incolore |
| 19" | dodécane pour 5 g de MR | 7,02 g | 26,3 | 3,1 | orange | 8,9 | incolore |
| 20 | octane | 39 g | 6,1 | 29,1 | orange | 41 | jaune |
| 21 | octane | 34 g | 5,9 | 13,8 | brun clair | 59,3 | incolore |
| 22 | octane | 35 g | 12,7 | 31,1 | jaune-vert | 41,2 | incolore |
| 23 | décane pour 10 g de MR | 36,5 g | 8,41 | 4,9 | jaune | 40,7 | incolore |

Tableau V D

| Ex | phases organiques n° | Quantités extraites dans les phases Organiques P 3% | C 9% | HC 8% | C 6% | Pd ppm |
|---|---|---|---|---|---|---|
| 9 a | 1 | 75 | | | | ≤4 |
| 10 | 1 | 69 | | 100 | 80 | 2,6 |
| | 2 | 88 | | 100 | 100 | 0,1 |
| 11 | 1 | 61 | 75 | 77 | | 10,6 |
| | 2 | 88 | 100 | 100 | | 0,7 |
| | 3 | 96 | 100 | 100 | | <0,1 |
| 12 | 1 | 97 | 100 | | 100 | <5 |
| 13 | 1 | | | | | <3 |
| 14 | 1 | 98 | 100 | 96 | 100 | 2,9 |
| 15 | 1 | 35 | 62 | 43 | | 17,5 |
| | 2 | 58 | 88 | 61 | | 2,1 |
| | 3 | 66 | 88 | 90 | | 0,1 |
| 16 | 1 | | | | | 20 |
| 17 | 1 | | | | | 8 |
| 18 | 1 | 85 | 81 | 69 | | 3,7 |
| 19 | 1 | | | | | 8 |
| 19′ | 1 | | | | | 15 |
| 19″ | 1 | | | | | 2 |
| 20 | 1 | | | | | 2,8 |
| 21 | 1 | | | | | 0,5 |
| 22 | 1 | | | | | 5 |
| 23 | 1 | | | | | <0,2 |

**Revendications**

1. Procédé de séparation du palladium des produits issus de la réaction de carbonylation par de l'oxyde de carbone des diènes conjugués en présence d'un alcool, d'un hydracide halogéné et d'un catalyseur au palladium, réaction de carbonylation en vue de la synthèse d'esters d'acides carboxyliques $\beta,\gamma$- insaturés, ledit procédé étant caractérisé en ce que:

- les produits issus de la réaction de carbonylation sont mis en contact avec un sel d'onium quaternaire d'un élément du groupe VB choisi parmi l'azote, le phosphore et l'arsenic, un alcool et un solvant apolaire hydrocarboné aliphatique ou cycloaliphatique présentant une constante diélectrique (mesurée à 20–25°C) inférieure à 2,3.
- et en ce qu'après décantation de la phase alcoolique et de la phase organique formées lors de l'opération de mise en contact, on récupère le palladium et le sel d'onium quaternaire dans la phase alcoolique et les produits issus de la réaction de carbonylation dans la phase organique.

2. Procédé selon la revendication 1, caractérisé en ce que le sel d'onium quaternaire présente un cation répondant à l'une des formules I à IV suivantes:

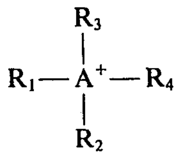

(I)

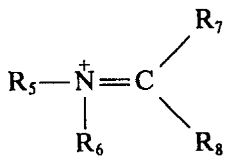

(II)

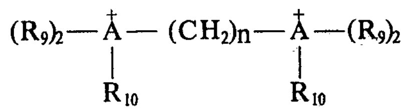

(III)

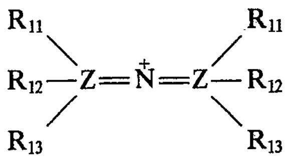

(IV)

où:

- A représente de l'azote, du phosphore ou de l'arsenic
- $R_1$, $R_2$, $R_3$, $R_4$ sont identiques ou différents et représentent:

  un radical alkyle linéaire ou ramifié contenant de 1 à 16 atomes de carbone, ledit radical alkyle étant éventuellement substitué par un groupe phényle, hydroxy, halogéno, nitro, alkoxy, ou alkoxycarbonyle;
  un radical alcényle linéaire ou ramifié contenant de 2 à 12 atomes de carbone;
  un radical aryle contenant de 6 à 10 atomes de carbone, ledit radical aryle étant éventuellement substitué par au moins un groupe alkyle contenant de 1 à 4 atomes de carbone, alcoxy, alkoxycarbonyle, ou halogéno;
  deux desdits radicaux $R_1$ à $R_4$ pouvant former ensemble un radical alkylène, alcénylène ou alcadiènylène, linéaire ou ramifié et contenant de 3 à 6 atomes de carbone;

- $R_5$, $R_6$, $R_7$, $R_8$ sont identiques ou différents et représentent:

  un radical alkyle linéaire ou ramifié contenant de 1 à 4 atomes de carbone;
  les radicaux $R_7$ et $R_8$ pouvant former ensemble un radical alkylène contenant de 3 à 6 atomes de carbone;
  les radicaux $R_6$ et $R_7$ ou $R_6$ et $R_8$ pouvant former ensemble un radical alkylène, alcénylène ou alcadiénylène, contenant 4 atomes de carbone et constituant avec N un hétérocycle azoté;

- $R_9$ représente un radical alkyle linéaire ou ramifié contenant de 1 à 4 atomes de carbone ou un radical phényle;
- $R_{10}$ représente:

  un radical alkyle linéaire ou ramifié contenant de 1 à 4 atomes de carbone, semblable à ou différent de $R_9$;
  un radical alcényle linéaire ou ramifié, contenant de 2 à 12 atomes de carbone;

- n représente un nombre entier supérieur ou égal à 1 et inférieur ou égal à 10;
- Z représente du phosphore ou de l'arsenic;
- $R_{11}$, $R_{12}$ et $R_{13}$ sont identiques ou différents et représentent

  un radical aryle contenant de 6 à 10 atomes de carbone.

3. Procédé selon la revendication 2, caractérisé en ce que lorsqu'ils représentent des radicaux alcényles, les groupes $R_1$, $R_2$, $R_3$, $R_4$ et $R_{10}$ contiennent de 4 à 8 atomes de carbone.
4. Procédé selon la revendication 3, caractérisé en ce que les radicaux $R_1$, $R_2$, $R_3$, $R_4$ et $R_{10}$ sont des radicaux alcényles dérivés du diène conjugué mis en oeuvre.

5. Procédé selon l'une quelconque des revendications 2 à 4, caractérisé en ce que n est un nombre entier inférieur ou égal à 6.

6. Procédé selon l'une quelconque des revendications 1 à 5, caractérisé en ce que ledit cation est un cation:

tétraméthylammonium,
tétraéthylammonium,
tétrabutylammonium,
tétradodécylammonium,
méthyltrioctylammonium,
hexadécyltriméthylammonium,
méthyltriphénylammonium,
benzyltriméthylammonium,
butène-2 yltriéthylammonium,
tétraméthylphosphonium,
tétrabutylphosphonium,
hexadécyltributylphosphonium,
éthyltriméthylphosphonium,
méthyltriphénylphosphonium,
hexadécylpyridinium,
bis(butène-2 yldiméthylammonium)-1,3 propane,
tétraphénylarsonium,
tétrakis (hydroxyméthyl)phosphonium,
tétraphénylphosphonium,
bis(triphénylphosphine)iminium.

7. Procédé selon l'une quelconque des revendications 1 à 6, caractérisé en ce que l'anion dudit sel d'onium est un anion

$H_2PO_4^-$ $F^-$ $ClO_4^-$ $PF_6^-$ $BF_4^-$ $B\Phi_4^-$ $PO_4^{3-}$ $HPO_4^{2-}$ $CH_3SO_3^-$

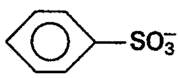 $HSO_4^-$ $NO_3^-$ $SO_4^{2-}$ $Cl^-$ $Br^-$ $I^-$ $SCN^-$ $CN^-$ $RS^-$

où R représente un radical alkyle en $C_1$—$C_6$ ou aryle en $C_6$—$C_{10}$.

8. Procédé selon la revendication 7, caractérisé en ce que l'anion est un des anions suivants:

$PO_4^{3-}$ $HPO_4^{2-}$ $H_2PO_4^-$ $CH_3SO_3^-$ 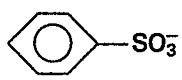 $NO_3^-$ $SO_4^{2-}$ $PF_6^-$ $Cl^-$

$Br^-$.

9. Procédé selon la revendication 8, caractérisé en ce que l'anion est un anion $Cl^-$.

10. Procédé selon l'une quelconque des revendications 1 à 9, caractérisé en ce que l'alcool mis en oeuvre est un monoalcool contenant de 1 à 4 atomes de carbone.

11. Procédé selon la revendication 10, caractérisé en ce que l'alcool mis en oeuvre est un monoalcool contenant de 1 à 3 atomes de carbone.

12. Procédé selon l'une quelconque des révendications 1 à 11, caractérisé en ce que le solvant apolaire présente une constante diélectrique inférieure à 2,1.

13. Procédé selon la revendication 12, caractérisé en ce que le solvant apolaire est l'un des solvants suivants: le pentane, l'isopentane, l'hexane, le cyclohexane, l'octane, le cyclooctane, le triméthyl-2, -2,4 pentane, le décane, le dodécane, le tétradécane, l'hexadécane ou leurs mélanges du type éther de pétrole.

14. Procédé selon l'une quelconque des revendications 1 à 13, caractérisé en ce que l'opération de mise en contact est réalisée en présence:

— d'une quantité de sel d'onium quaternaire correspondant à un rapport molaire cation onium quaternaire/palladium d'au moins 10;
— et des quantités d'alcool et de solvant hydrocarboné apolaire au moins égales à celles nécessaires à la décantation du milieu de mise en contact en une phase alcoolique et en une phase organique.

15. Procédé selon la revendication 14, caractérisé en ce que l'opération de mise en contact est réalisée en présence:

— d'une quantité d'alcool correspondant à un rapport pondéral sel d'onium quaternaire/alcool d'au moins 0,1;

– d'une quantité de solvant correspondant à un rapport pondéral solvant/alcool supérieur à 1.

16. Procédé selon la revendication 14 ou 15, caractérisé en ce que l'opération de mise en contact est réalisée en présence:

– d'une quantité de sel d'onium quaternaire correspondant à un rapport molaire cation onium/palladium compris entre 20 et 200;
– d'une quantité d'alcool correspondant à un rapport pondéral sel d'onium quaternaire/alcool compris entre 0,25 et 30;
– d'une quantité de solvant correspondant à un rapport pondéral solvant/alcool supérieur à 1,5.

17. Premier mode de réalisation du procédé faisant l'objet de l'une quelconque des revendications 1 à 16, caractérisé en ce que:

– le milieu issu de l'opération de carbonylation contenant éventuellement de l'alcool non transformé, est additionné d'un sel d'onium quaternaire, d'un solvant hydrocarboné apolaire et éventuellement d'alcool, les quantités de solvant et d'alcool étant ajustées de façon à obtenir une séparation de la phase alcoolique et de la phase organique après décantation;
– l'on récupère la phase alcoolique contenant l'alcool, le sel d'onium quaternaire et le catalyseur au palladium.

18. Deuxième mode de réalisation du procédé faisant l'objet de l'une quelconque des revendications 1 à 6 et 8 à 16, caractérisé en ce que:

– le sel d'onium quaternaire est choisi parmi ceux dont l'anion est une base »dure« ou »intermédiaire«;
– ledit sel d'onium quaternaire est mis en oeuvre en totalité ou en partie au cours de la réaction de carbonylation;
– le milieu issu de l'opération de carbonylation contenant le sel d'onium et éventuellement de l'alcool non réagi est additionné de sel d'onium si nécessaire, d'un solvant hydrocarboné apolaire et éventuellement d'alcool, les quantités de solvant et d'alcool étant ajustées de façon à obtenir une séparation de la phase alcoolique et de la phase organique après décantation;
– l'on récupère la phase alcoolique contenant l'alcool, le sel d'onium quaternaire et le catalyseur au palladium.

19. Deuxième mode de réalisation selon la revendication 18, caractérisé en ce que la quantité de sel d'onium quaternaire mise en oeuvre au cours la réaction de carbonylation correspond à un rapport molaire cation onium/Pd supérieur à 0,5.

20. Deuxième mode de réalisation selon la revendication 19, caractérisé en ce que la quantité de sel d'onium quaternaire mise en oeuvre au cours de la réaction de carbonylation correspond à un rapport molaire cation onium/Pd compris entre 1 et 15.

21. Modes de réalisation selon l'une quelconque des revendications 17 à 20, caractérisé en ce que le solvant apolaire est introduit en tout ou partie au cours de la réaction de carbonylation.

22. Procédé selon l'une quelconque des revendications 1 et 17 à 21, caractérisé en ce que la phase alcoolique obtenue après décantation est soumise à une ou plusieurs opérations d'extraction et de séparation à l'aide dudit solvant hydrocarboné apolaire.

**Patentansprüche**

1. Verfahren zur Abtrennung von Palladium von den Produkten, die bei der Carbonylierungsreaktion von konjugierten Dienen mit Hilfe von Kohlenoxid in Gegenwart eines Alkohols, einer Halogenwasserstoffsäure und eines Palladiumkatalysators entstehen, wobei die Carbonylierungsreaktion durchgeführt wird zur Herstellung von Estern von $\beta,\gamma$-ungesättigten Carbonsäuren, dadurch gekennzeichnet, daß

– die bei der Carbonylierungsreaktion entstandenen Produkte mit einem quaternären Oniumsalz eines Elements der Gruppe VB, ausgewählt aus Stickstoff, Phosphor und Arsen, einem Alkohol und einem apolaren aliphatischen oder cycloaliphatischen Kohlenwasserstoff als Lösungsmittel, mit einer Dielektrizitätskonstante (gemessen bei 20 bis 25°C) unter 2,3 zusammengebracht werden,
– und daß man nach dem Trennen der alkoholischen Phase und der organischen Phase durch Absetzen, die beim Zusammenbringen entstanden sind, das Palladium und das quaternäre Oniumsalz aus der alkoholischen Phase und die Produkte der Carbonylierungsreaktion aus der organischen Phase gewinnt.

2. Verfahren nach Anspruch 1, dadurch gekennzeichnet, daß das quaternäre Oniumsalz ein Kation aufweist, entsprechend einer der folgenden Formeln I bis IV:

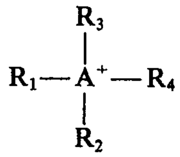

(I)

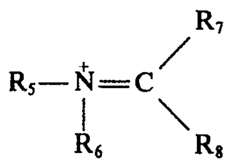

(II)

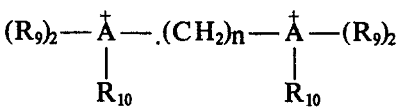

(III)

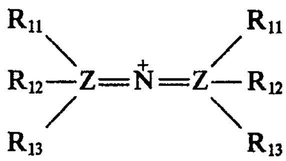

(IV)

bei denen

- A Stickstoff, Phosphor oder Arsen bedeutet,
- $R_1$, $R_2$, $R_3$ und $R_4$ gleich oder verschieden sind und bedeuten:

  einen linearen oder verzweigten Alkylrest mit 1 bis 16 Kohlenstoffatomen, der gegebenenfalls substituiert ist durch ein Halogenatom, eine Phenyl-, Hydroxy-, Nitro-, Alkoxy- oder Alkoxycarbonylgruppe,
  einen linearen oder verzweigten Alkenylrest mit 2 bis 12 Kohlenstoffatomen,
  einen Arylrest mit 6 bis 10 Kohlenstoffatomen, der gegebenenfalls substituiert ist, durch mindestens eine Alkylgruppe mit 1 bis 4 Kohlenstoffatomen, Alkoxygruppe, Alkoxycarbonylgruppe oder Halogenatom,
  zwei der Reste $R_1$ bis $R_4$ zusammen einen linearen oder verzweigten Alkylen-, Alkenylen- oder Alkadienylenrest mit 3 bis 6 Kohlenstoffatomen bilden können,

- $R_5$, $R_6$, $R_7$ und $R_8$ gleich oder verschieden sind und bedeuten:

  einen linearen oder verzweigten Alkylrest mit 1 bis 4 Kohlenstoffatomen,
  die Reste $R_7$ und $R_8$ zusammen einen Alkylenrest mit 3 bis 6 Kohlenstoffatomen bilden können,
  die Reste $R_6$ und $R_7$ oder $R_6$ und $R_8$ zusammen einen Alkylen; Alkenylen- oder Alkadienylenrest mit 4 Kohlenstoffatomen bilden können, der zusammen mit N einen Stickstoff-Heterocyclus ergibt,

- $R_9$ einen linearen oder verzweigten Alkylrest mit 1 bis 4 Kohlenstoffatomen oder einen Phenylrest bedeutet,
- $R_{10}$ bedeutet:

  einen linearen oder verzweigten Alkylrest mit 1 bis 4 Kohlenstoffatomen, gleich oder verschieden von $R_9$,
  einen linearen oder verzweigten Alkenylrest mit 2 bis 12 Kohlenstoffatomen,

- n eine ganze Zahl größer oder gleich 1 und kleiner oder gleich 10 bedeutet,
- Z Phosphor oder Arsen bedeutet,
- $R_{11}$, $R_{12}$ und $R_{13}$ gleich oder verschieden sind und bedeuten:

  einen linearen oder verzweigten Alkylrest mit 1 bis 16 Kohlenstoffatomen,
  einen Arylrest mit 6 bis 10 Kohlenstoffatomen.

3. Verfahren nach Anspruch 2, dadurch gekennzeichnet, daß die Reste $R_1$, $R_2$, $R_3$, $R_4$ und $R_{10}$, wenn sie Alkenylgruppen bedeuten, 4 bis 8 Kohlenstoffatome aufweisen.

4. Verfahren nach Anspruch 3, dadurch gekennzeichnet, daß die Reste $R_1$, $R_2$, $R_3$, $R_4$ und $R_{10}$ Alkenylreste sind, die abgeleitet sind von dem eingesetzten konjugierten Dien.

5. Verfahren nach einem der Ansprüche 2 bis 4, dadurch gekennzeichnet, daß n eine ganze Zahl kleiner gleich 6 ist.

6. Verfahren nach einem der Ansprüche 1 bis 5, dadurch gekennzeichnet, daß das Kation eines der folgenden Kationen ist:

Tetramethylammonium,
Tetraethylammonium,
Tetrabutylammonium,
Tetradodecylammonium,
Methyltrioctylammonium,
Hexadecyltrimethylammonium,
Methyltriphenylammonium,
Benzyltrimethylammonium,
2-Butenyltriethylammonium,
Tetramethylphosphonium,
Tetrabutylphosphonium,
Hexadecyltributylphosphonium,
Ethyltrimethylphosphonium,
Methyltriphenylphosphonium,
Hexadecylpyridinium,
1,3-Bis-(2-butenyldimethylammonium)-propan,
Tetraphenylarsonium,
Tetrakis(hydroxymethyl)phosphonium,
Tetraphenylphosphonium,
Bis(triphenylphosphin)iminium.

7. Verfahren nach einem der Ansprüche 1 bis 6, dadurch gekennzeichnet, daß das Anion dieses Oniumsalzes eines der folgenden Anionen ist:

$H_2PO_4^-$ $F^-$ $ClO_4^-$ $PF_6^-$ $BF_4^-$ 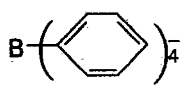 $PO_4^{3-}$ $HPO_4^{2-}$ $CH_3SO_3^-$

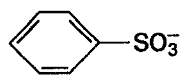 $HSO_4^-$ $NO_3^-$ $SO_4^{2-}$ $Cl^-$ $Br^-$ $I^-$ $SCN^-$ $CN^-$ $RS^-$

wobei R einen $C_1$- bis $C_6$-Alkyl- oder einen $C_6$- bis $C_{10}$-Arylrest bedeutet.

8. Verfahren nach Anspruch 7, dadurch gekennzeichnet, daß das Anion eines der folgenden Anionen ist:

$PO_4^{3-}$ $HPO_4^{2-}$ $H_2PO_4^-$ $CH_3SO_3^-$ 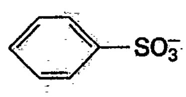 $NO_3^-$ $SO_4^{2-}$ $PF_6^-$ $Cl^-$

$Br^-$.

9. Verfahren nach Anspruch 8, dadurch gekennzeichnet, daß das Anion ein $Cl^-$-Anion ist.

10. Verfahren nach einem der Ansprüche 1 bis 9, dadurch gekennzeichnet, daß der eingesetzte Alkohol ein einwertiger Alkohol mit 1 bis 4 Kohlenstoffatomen ist.

11. Verfahren nach Anspruch 10, dadurch gekennzeichnet, daß der eingesetzte Alkohol ein einwertiger Alkohol mit 1 bis 3 Kohlenstoffatomen ist.

12. Verfahren nach einem der Ansprüche 1 bis 11, dadurch gekennzeichnet, daß das apolare Lösungsmittel eine Dielektrizitätskonstante unter 2,1 aufweist.

13. Verfahren nach Anspruch 12, dadurch gekennzeichnet, daß das apolare Lösungsmittel eines der folgenden Lösungsmittel ist: Pentan, Isopentan, Hexan, Cyclohexan, Octan, Cyclooctan, 2,2,4-Trimethylpentan, Decan, Dodecan, Tetradecan, Hexadecan oder deren Petrolether-artigen Gemische.

14. Verfahren nach einem der Ansprüche 1 bis 13, dadurch gekennzeichnet, daß das Zusammenbringen durchgeführt wird in Gegenwart von:

- einer Menge des quaternären Oniumsalzes, entsprechend einem Molverhältnis von Kation des quaternären Oniumsalzes zu Palladium von mindestens 10,
- und von solchen Mengen an Alkohol und an apolarem Kohlenwasserstofflösungsmittel, die zumindest gleich sind denjenigen, die erforderlich sind zur Auftrennung des Mediums in eine alkoholische Phase und eine organische Phase durch Absetzen.

15. Verfahren nach Anspruch 14, dadurch gekennzeichnet, daß das Zusammenbringen durchgeführt wird in Gegenwart von:

- einer Menge Alkohol, die einem Gewichtsverhältnis von quaternärem Oniumsalz zu Alkohol von zumindest 0,1 entspricht,
- einer Menge an Lösungsmittel, entsprechend einem Gewichtsverhältnis Lösungsmittel zu Alkohol über 1.

16. Verfahren nach Anspruch 14 oder 15, dadurch gekennzeichnet, daß das Zusammenbringen durchgeführt wird in Gegenwart von:

- einer Menge an quaternärem Oniumsalz, entsprechend einem Molverhältnis Oniumkation zu Palladium zwischen 20 und 200,
- einer Menge an Alkohol, entsprechend einem Gewichtsverhältnis quaternäres Oniumsalz zu Alkohol zwischen 0,25 und 30,
- einer Menge an Lösungsmittel, entsprechend einem Gewichtsverhältnis Lösungsmittel zu Alkohol über 1,5.

17. Erste Ausführungsform des Verfahrens nach einem der Ansprüche 1 bis 16, dadurch gekennzeichnet, daß

- zu dem bei der Carbonylierungsreaktion entstandenen Gemisch, das gegebenenfalls nicht umgesetzten Alkohol enthält, ein quaternäres Oniumsalz, ein apolares Kohlenwasserstofflösungsmittel und gegebenenfalls Alkohol zugegeben wird, wobei die Mengen an Lösungsmittel und Alkohol so eingestellt werden, daß man eine Trennung der alkoholischen Phase von der organischen Phase nach dem Absetzen erhält,
- man die alkoholische Phase, enthaltend den Alkohol, das quaternäre Oniumsalz und den Palladiumkatalysator, gewinnt.

18. Zweite Ausführungsform des Verfahrens nach einem der Ansprüche 1 bis 6 und 8 bis 16, dadurch gekennzeichnet, daß

- das quaternäre Oniumsalz ausgewählt wird aus solchen, deren Anion eine »harte« oder »mittlere« Base ist,
- das quaternäre Oniumsalz auf einmal oder in Anteilen während der Carbonylierungsreaktion zugegeben wird,
- zu dem bei der Carbonylierungsreaktion entstandenen Gemisch, enthaltend das Oniumsalz und gegebenenfalls den nicht umgesetzten Alkohol, das Oniumsalz soweit erforderlich, ein apolares Kohlenwasserstofflösungsmittel und gegebenenfalls Alkohol zugesetzt wird, wobei die Mengen an Lösungsmittel und Alkohol so eingestellt werden, daß man eine Trennung der alkoholischen Phase und der organischen Phase nach dem Absetzen erhält,
- man die den Alkohol, das quaternäre Oniumsalz und den Palladiumkatalysator enthaltende alkoholische Phase gewinnt.

19. Zweite Ausführungsform nach Anspruch 18, dadurch gekennzeichnet, daß die Menge an quaternärem Oniumsalz, die während der Carbonylierungsreaktion zugesetzt wird, einem Molverhältnis von Oniumkation zu Palladium über 0,5 entspricht.

20. Zweite Ausführungsform nach Anspruch 19, dadurch gekennzeichnet, daß die im Verlauf der Carbonylierungsreaktion eingesetzte Menge an quaternärem Oniumsalz einem Molverhältnis Oniumkation zu Pd zwischen 1 und 15 entspricht.

21. Ausführungsform nach einem der Ansprüche 17 bis 20, dadurch gekennzeichnet, daß das polare Lösungsmittel im Ganzen oder in Anteilen im Laufe der Carbonylierungsreaktion zugegeben wird.

22. Verfahren nach einem der Ansprüche 1 und 17 bis 21, dadurch gekennzeichnet, daß die nach dem Absetzen erhaltene alkoholische Phase einer oder mehreren Extraktions- und Trennungsoperationen mit Hilfe des apolaren Kohlenwasserstofflösungsmittels unterworfen wird.

**Claims**

1. Process for separating palladium from the products originating from the reaction of carbonylation of conjugated dienes with carbon monoxide in the presence of an alcohol, a hydrogen halide and a palladium catalyst, a carbonylation reaction with a view to the synthesis of esters of $\beta,\gamma$-unsaturated carboxylic acids, the said process being characterised in that:

– the products originating from the carbonylation reaction are placed in contact with a quaternary onium salt of a VB group element chosen from nitrogen, phosphorus and arsenic, an alcohol and a nonpolar aliphatic or cycloaliphatic hydrocarbon solvent having a dielectric constant (measured at 20–25°C) below 2.3,
– and in that after decanting of the alcohol phase and of the organic phase which are formed during the operation of placing in contact, the palladium and the quaternary onium salt in the alcoholic phase and the products originating from the carbonylation reaction in the organic phase are recovered.

2. Process according to Claim 1, characterised in that the quaternary onium salt has a cation corresponding to one of the following formulae I to IV:

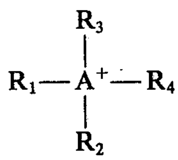
(I)

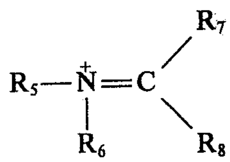
(II)

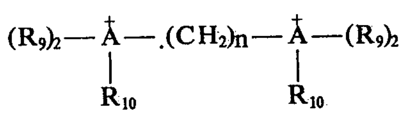
(III)

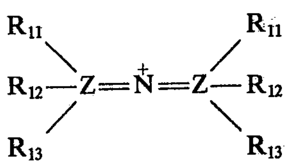
(IV)

where:

– A denotes nitrogen, phosphorus or arsenic
– $R_1$, $R_2$, $R_3$ and $R_4$ are identical or different and denote:

a linear or branched alkyl radical containing from 1 to 16 carbon atoms, the said alkyl radical being optionally substituted by a phenyl, hydroxy, halo, nitro, alkoxy or alkoxycarbonyl group;
a linear or branched alkenyl radical containing from 2 to 12 carbon atoms;
an aryl radical containing from 6 to 10 carbon atoms, the said aryl radical being optionally substituted by at least one alkyl group containing from 1 to 4 carbon atoms, an alkoxy, alkoxycarbonyl or halo group;
two of the said radicals $R_1$ to $R_4$ being capable of together forming an alkylene, alkenylene or alkadienylene radical, linear or branched and containing from 3 to 6 carbon atoms;

– $R_5$, $R_6$, $R_7$ and $R_8$ are identical or different and denote:

a linear or branched alkyl radical containing from 1 to 4 carbon atoms;
the radicals $R_7$ and $R_8$ capable of forming together an alkylene radical containing from 3 to 6 carbon atoms;
the radicals $R_6$ and $R_7$ or $R_6$ and $R_8$ capable of forming together an alkylene, alkenylene or alkadienylene radical containing 4 carbon atoms and forming with N a nitrogenous heterocyclic ring;

– $R_9$ denotes a linear or branched alkyl radical containing from 1 to 4 carbon atoms or a phenyl radical;
– $R_{10}$ denotes:

a linear or branched alkyl radical containing from 1 to 4 carbon atoms, similar to or different from $R_9$;
a linear or branched alkenyl radical containing from 2 to 12 carbon atoms;

- n denotes an integer greater than or equal to 1 and smaller than or equal to 10;
- Z denotes phosphorus or arsenic;
- $R_{11}$, $R_{12}$ and $R_{13}$ are identical or different and denote:

  a linear or branched alkyl radical containing from 1 to 16 carbon atoms;
  an aryl radical containing from 6 to 10 carbon atoms.

3. Process according to Claim 2, characterised in that, when they denote alkenyl radicals, the groups $R_1$, $R_2$, $R_3$, $R_4$ and $R_{10}$ contain from 4 to 8 carbon atoms.

4. Process according to Claim 3, characterised in that the radicals $R_1$, $R_2$, $R_3$, $R_4$ and $R_{10}$ are alkenyl radicals derived from the conjugated diene employed.

5. Process according to any one of Claims 2 to 4, characterised in that n is an integer smaller than or equal to 6.

6. Process according to any one of Claims 1 to 5, characterised in that the said cation is a:

tetramethylammonium,
tetraethylammonium,
tetrabutylammonium,
tetradodecylammonium,
methyltrioctylammonium,
hexadecyltrimethylammonium,
methyltriphenylammonium,
benzyltrimethylammonium,
buten-2-yltriethylammonium,
tetramethylphosphonium,
tetrabutylphosphonium,
hexadecyltributylphosphonium,
ethyltrimethylphosphonium,
methyltriphenylphosphonium,
hexadecylpyridinium,
bis-(buten-2-yldimethylammonium)-1,3-propane,
tetraphenylarsonium,
tetrakis(hydroxymethyl)phosphonium,
tetraphenylphosphonium, or
bis(triphenylphosphine)iminium

cation.

7. Process according to any one of Claims 1 to 6, characterised in that the anion of the said onium salt is a

$H_2PO_4^-$ $F^-$ $ClO_4^-$ $PF_6^-$ $BF_4^-$ $B\Phi_4^-$ $PO_4^{3-}$ $HPO_4^{2-}$ $CH_3SO_3^-$

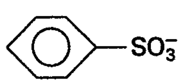 $HSO_4^-$ $NO_3^-$ $SO_4^{2-}$ $Cl^-$ $Br^-$ $I^-$ $SCN^-$

$CN^-$ or $RS^-$

anion, where R denotes a $C_1$—$C_6$ alkyl radical or an $C_6$—$C_{10}$ aryl radical.

8. Process according to Claim 7, characterised in that the anion is one of the following anions:

$PO_4^{3-}$ $HPO_4^{2-}$ $H_2PO_4^-$ $CH_3SO_3^-$ 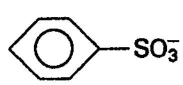 $NO_3^-$ $SO_4^{2-}$ $PF_6^-$ $Cl^-$

or $Br^-$.

9. Process according to Claim 8, characterised in that the anion is a $Cl^-$ anion.

10. Process according to any one of Claims 1 to 9, characterised in that alcohol employed is a monoalcohol containing from 1 to 4 carbon atoms.

11. Process according to Claim 10, characterised in that the alcohol employed is a monoalcohol containing from 1 to 3 carbon atoms.

12. Process according to any one of Claims 1 to 11, characterised in that the nonpolar solvent has a dielectric constant lower than 2.1.

13. Process according to Claim 12, characterised in that the nonpolar solvent is one of the following solvents: pentane, isopentane, hexane, cyclohexane, octane, cyclooctane, 2,2,4-trimethylpentane, decane, dodecane, tetradecane, hexadecane or their mixtures of the petroleum ether type.

14. Process according to any one of Claims 1 to 13, characterised in that the operation of placing in contact is carried out in the presence:

- of a quantity of a quaternary onium salt corresponding to a quaternary onium cation/palladium molar ratio of at least 10;
- and of quantities of alcohol and nonpolar hydrocarbon solvent which are at least equal to those necessary for the decanting of the medium for placing in contact as an alcoholic phase and an organic phase.

15. Process according to Claim 14, characterised in that the operation of placing in contact is carried out in the presence:

- of a quantity of alcohol corresponding to a quaternary onium salt/alcohol weight ratio of at least 0.1;
- of a quantity of solvent corresponding to a solvent/alcohol weight ratio greater than 1.

16. Process according to Claim 14 or 15, characterised in that the operation of placing in contact is carried out in the presence:

- of a quantity of quaternary onium salt corresponding to an onium cation/palladium molar ratio of between 20 and 200;
- of a quantity of alcohol corresponding to a quaternary onium salt/alcohol weight ratio of between 0.25 and 30;
- of a quantity of solvent corresponding to a solvent/alcohol weight ratio greater than 1.5.

17. First embodiment of the process forming the subject of any one of Claims 1 to 16, characterised in that:

- a quaternary onium salt, a nonpolar hydrocarbon solvent and, if appropriate, alcohol are added to the medium originating from the carbonylation operation, possibly containing unconverted alcohol, the quantities of solvent and alcohol being adjusted so as to obtain a separation of the alcoholic phase from the organic phase after decantation;
- the alcoholic phase containing the alcohol, the quaternary onium salt and the palladium-based catalyst are recovered.

18. Second embodiment of the process forming the subject of any one of Claims 1 to 6 and 8 to 16, characterised in that:

- the quaternary onium salt is chosen from those in which the anion is a »hard« or »intermediate« base;
- the said quaternary onium salt is employed wholly or partly during the carbonylation reaction;
- an onium salt if necessary, a nonpolar hydrocarbon solvent and, if appropriate, alcohol are added to the medium originating from the carbonylation operation containing the onium salt and possibly unreacted alcohol, the quantities of solvent and alcohol being adjusted so as to obtain a separation of the alcoholic phase from the organic phase after decantation;
- the alcoholic phase containing the alcohol, the quaternary onium salt and the palladium-based catalyst are recovered.

19. Second embodiment according to Claim 18, characterised in that the quantity of quaternary onium salt employed during the carbonylation reaction corresponds to an onium cation/Pd molar ratio greater than 0.5.

20. Second embodiment according to Claim 19, characterised in that the quantity of quaternary onium salt employed during the carbonylation reaction corresponds to an onium cation/Pd molar ratio of between 1 and 15.

21. Embodiments according to any one of Claims 17 to 20, characterised in that the nonpolar solvent is introduced wholly or partly during the carbonylation reaction.

22. Process according to any one of Claims 1 and 17 to 21, characterised in that the alcoholic phase obtained after decantation is subjected to one or more extraction and separation operations with the aid of the said nonpolar hydrocarbon solvent.